# EUROPEAN PATENT SPECIFICATION

(11) **EP 4 172 114 B1**
(45) Date of publication and mention of the grant of the patent: **01.05.2024**
(21) Application number: 21772724.7
(22) Date of filing: 30.08.2021
(51) Int. Cl.: C02F 3/08, C02F 3/10, B01J 19/30, G01N 11/16, C08L 23/04, C12N 11/082, C12M 1/12

(54) **USE OF A BIOFILM CARRIER FOR MOVING BED BIOFILM REACTORS**
VERWENDUNG EINES BIOFILMTRÄGERS FÜR WANDERBETT BIOFILM-REAKTOREN
UTILISATION D'UN SUPPORT DE BIOFILM POUR RÉACTEURS À BIOFILM À LIT MOBILE

(30) Priority: 31.08.2020 SE 2051008
(43) Date of publication of application: 03.05.2023
(73) Proprietor: Veolia Water Solutions & Technologies Support, 94417 Saint-Maurice Cedex (FR)
(72) Inventor: MAGNUSSON, Per, 211 31 Malmö (SE)
(74) Representative: Ström & Gulliksson AB
(86) International application number: PCT/EP2021/073882
(87) International publication number: WO 2022/043550

(56) References cited:
- EP-A1- 0 776 863
- EP-A1- 1 464 624
- EP-A1- 1 790 580
- US-A1- 2010 210 791
- US-A1- 2016 376 175
- US-A1- 2020 024 376

## Description

### Field of the Invention

This invention pertains in general to the field of moving bed biofilm reactors using carriers. More particularly the invention relates to the use of carrier elements for growth of biofilm thereon, wherein said carrier elements are designed to flow freely in a liquid to be purified from contaminants biologically by said growing biofilm. Furthermore, said carrier elements being further designed to have a significantly improved lifetime in said purification process compared to state of the art carrier elements of said use.

### Background of the Invention

It is known that in biological treatment of water or wastewater, the water is passed through some type of reactor or several reactors (a vessel or another space) wherein micro-organisms are utilized for converting pollutants in the water to harmless end products such as carbon dioxide and water. The treatment can be performed under supply of air (aerobically), without supply of air (anaerobically) or without supply of air but with presence of significant amounts of nitrate (anoxically).

In order to increase the efficiency of the treatment process, it is common to aim at a high content of active microorganisms in the process by preventing such organisms to escape together with the treated water, either by allowing the micro-organisms to grow suspended in the reactor and separating them from the water in a separation stage after the reactor and returning the micro-organisms to the reactor (e.g. the activated sludge process), or by introducing some kind of support material having surfaces into the process reactor on which the microorganisms can grow as a biofilm and thus be retained in the process (the biofilm process).

There are also mixtures of these two process types, referred to as hybrid processes, wherein the support material is introduced into the activated sludge process so that suspended microorganisms as well as biofilm growing microorganisms can be utilized in the process. The biofilm process has several advantages compared to the activated sludge process. For example, higher loads can be applied and the biofilm processes are substantially less sensitive to variations and disturbances. Many conventional biofilm processes are based on packing of carrier material in the treatment reactor, said material comprising fill bodies or blocks which are maintained fixed and immovable in the process. These embodiments of the process involve the risk of clogging of the biofilm bed by biomass or another particulate material and formation of dead zones in the process, wherein the contact between the water and the active microorganisms is unsatisfactory.

A type of biofilm process for wastewater treatment, which has become very successful during the last 25 years, is referred to as the MBBR process, i.e. "Moving Bed Biofilm Reactor" where a carrier material which kept in suspension and in movement in the process reactor volume is utilized. The carrier material having microorganisms growing thereon is maintained in the process by passing outgoing water through a strainer (sieve or grid) having an aperture diameter or slot width which is so small that the carrier material cannot pass through while the treated water does. The advantage of this kind of process is that the risk of clogging the biofilm bed and formation of dead zones is eliminated. The use of a carrier material which is kept in suspension and movement in the process was originally reported for different hybrid process applications, i.e. suspended carriers were supplied to activated sludge processes in order to improve the function thereof.

In moving bed reactors, the biofilm grows on a carrier that floats freely around in the reactor. The carrier material has generally either been foam rubber pieces or small pieces of plastic. Processes that use foam rubber pieces are known by the names Captor and Linpor. The disadvantages with foam rubber pieces are that the effective biofilm area is small because the growth on the outside of the foam rubber pieces clogs up the pores and prevents ingress of substrate and oxygen to the inner parts of the foam rubber pieces. Furthermore, one must use sieves that prevent the foam rubber pieces from leaving the reactors and one must have a system which regularly pumps the foam rubber pieces away from the sieves to prevent these from blocking up. Therefore, very few plants have been built with foam rubber as the carrier material.

However, nowadays many purification plants are built with moving bed processes where the carrier material is small pieces of plastic. The pieces of plastic are normally distributed evenly in the whole of the water volume and in practice one operates with degrees of filling with biofilm carrier medium all the way up to about 90% of the reactor volume. Sieves keep the plastic pieces in place in the reactor. The reactors are operated continuously without the need for back flushing. The process is very flexible with regard to the shape of the bioreactor. The specific biofilm surface area is higher than for trickling filters, but considerably smaller than in biological aerated filter (BAF) processes. However, on a total volume basis moving bed processes with a carrier material of small plastic pieces have been found to be as efficient as BAF processes when one takes into account the extra volume one needs for expansion of the filter bed and for the flushing water reservoir in the BAF processes. Examples of suppliers of moving bed processes with small plastic pieces as a carrier material are Veolia Water Technologies, Infilco, Degremont, Biowater Technology, and Aqwise systems.

Since the carriers in the MBBR process are exposed to repeated collisions with each other and other surfaces in the reactor such as reactor walls, submersed mixers, sieves and yet other equipment in the reactor, surfaces that are exposed to other carriers or other surfaces in the reactor are kept clean from biofilm growth. The efficiency of the process therefore is highly dependent on the area that is protected against collisions, for example in inner passages or compartments in the carriers. In fact, this protected surface area of the carrier media and the ability of the plastic pieces to by collisions with each other and other inner reactor surfaces clean each other from excess biofilm formation and thereby eliminating clogging and dead volumes is what makes the MBBR process with plastic media so efficient and effective.

Plastic carrier media for MBBR applications need to withstand harsh environments during extended periods of time. When used in MBBR plants world-wide for wastewater treatment they need to withstand among other things concentrated and constant mixing shear forces, shear forces from wall scraping, seasonal temperature changes and all kinds of chemicals both present in the wastewater and used in the wastewater treatment process for extended time periods in an aqueous media. The environment could become even harsher if the reactor wall conditions are poor (for example torn and rough walls) or for utilization of high speed mixers, high aeration rates (thereby higher shear forces and more collisions) or a high degree of carrier filling compared to reactor volume (thereby more collisions). These environments with time will ultimately make the plastic carrier pieces break and reach their end lifetime. Breakage of plastic carriers would not make it possible for them to work as biofilm carriers in MBBR. Naturally every wastewater treatment plant (WWTP) would like an as long carrier media lifetime as possible since it comes at cost to replace them with new plastic carrier pieces which is necessary for the biological wastewater treatment process to treat wastewater. Additionally, if carriers are not replaced in time before breakage, broken media pieces may go through the sieves and cause significant problems downstream in the WWTP and eventually leave the plant and spread into nature.

The lifetime of the state of the art employed plastic carrier media may vary widely in light of the different environmental conditions at different wastewater treatment plants as described above and if the conditions are rougher the lifetime will be shorter but could vary between, but not limited to, 5-25 years dependent on conditions. At the end of the state of the art carrier lifetime the MBBR tank walls and older worn equipment may have to be refurbished or replaced at great cost to reduce shear and friction when suspending new state of the art plastic carrier media to the reactor since the lifetime of the new media would otherwise be cut significantly shorter than when then plant was first built. US 2016/376175 A1 and EP 0 776 863 A1 disclose moving bed biofilm reactors (MBBR) using carriers made of polyethylene. US 2020/024376 A1 and EP 1 790 580 A1 disclose articles made of high density polyethylene having bimodal molecular weight distribution.

As such, there is a need for a carrier with greater lifetime, especially one that can work even in older or worn equipment.

### Summary of the Invention

Accordingly, the present invention preferably seeks to mitigate, alleviate or eliminate one or more of the above-identified deficiencies in the art and disadvantages singly or in any combination and solves at least the above mentioned problems by providing the use of a carrier to carry a biofilm in a moving bed biofilm reactor (MBBR) according to claim 1. The carrier having improved stability against environmental stresses, characterized in that the carrier comprises a carrier material, wherein the carrier material comprises at least one high density polyethylene having a bimodal molecular weight distribution, whereby the carrier material has a bimodal or a multimodal molecular weight distribution.

The present invention has the advantage over the prior art that the carrier to carry a biofilm in a moving bed biofilm reactor (MBBR) having improved stability against environmental stresses.

### Brief Description of the Drawings

These and other aspects, features and advantages of which the invention is capable of will be apparent and elucidated from the following description of embodiments of the present invention, reference being made to the accompanying drawings, in which
Fig. 1 is a representation of molecular structures for the most common kinds of polyethylene, high-density polyethylene (HDPE), linear low density polyethylene (LLDPE) and low density polyethylene (LDPE),
Fig. 2 is an illustration of the strain response from a tensile ductile deformation of a semi-crystalline polymer during stress application,
Fig. 3 is an illustration of the stages of brittle fracture; (a) crystalline lamellae starts to pull away, (b) the tie-molecules start to stretch tight, (c) clean break of lamellae,
Fig. 4 is an illustration showing the failure modes of a pipe made out of semi-crystalline polymer - internal hydrostatic pressure testing,
Fig. 5 is a graph showing the creep rupture curve,
Fig. 6 is an illustration showing the dependency of molecular weight and molecular weight distribution of the storage and loss modulus cross over point as function of angular frequency,
Fig. 7 is a graph showing the storage and loss modulus cross over point of end of life Media-1, end of life Media-2 and virgin media before use as function of angular frequency in a rheology frequency sweep at 180 °C,
Fig. 8 is a light microscopy image of an unused virgin Media-1 piece to the left and to the right a non-broken part of a MBBR utilized Media-1 piece,
Fig. 9 is a light microscopy "face-up" image of an unused virgin carrier Media-1 piece to the left and to the right a broken carrier Media-1 piece,
Fig. 10 is a light microscopy "side" image of an unused virgin carrier Media-1 piece to the left and to the right a broken carrier Media-1 piece Fig. 11 is a light microscopy "side" image of a damaged carrier Media-1 piece,
Fig. 12 is a light microscopy "side" image of a damaged carrier Media-1 piece,
Fig. 13 is a light microscopy "side" image of a damaged carrier Media-1 piece,
Fig. 14 is a light microscopy "side" image of a damaged carrier Media-1 piece,
Fig. 15 is a light microscopy "face-up" image of an unused virgin carrier Media-2 piece to the left and to the right a utilized carrier Media-2 piece,
Fig. 16 is a light microscopy "side" image of an unused virgin carrier Media-2 piece to the left and to the right a damaged carrier Media-2 piece,
Fig. 17 is a light microscopy "side" image of a damaged carrier Media-2 piece,
Fig. 18 is a light microscopy "side" image of an unused virgin carrier Media-2 piece to the left and to the right a damaged carrier Media-2 piece,
Fig. 19 is a light microscopy "side" image of a damaged carrier Media-2 piece,
Fig. 20 is a light microscopy "side" image of a damaged carrier Media-2 piece,
Fig. 21 is a light microscopy "side" image of a damaged carrier Media-2 piece,
Fig. 22 is a light microscopy "face-up" image of a broken carrier Media-2 piece,
Fig. 23 shows three graphs showing typical MWD with comonomer composition/SCB distribution for (a) polyethylene produced using Ziegler-Natta catalyst, (b) polyethylene produced using Ziegler-Natta catalyst in tandem process, (c) polyethylene produced using metallocene catalyst,
Fig. 24 shows a graph of molecular weight distribution of most synthetic polymers,
Fig. 25 is an illustration of the bimodal molecular weight distribution consisting of a high molecular weight copolymer giving increased stress cracking resistance and a low molecular weight homopolymer giving improved processability and higher stiffness,
Fig. 26 is a picture of the mixing blade used in the heavy mixing tests,
Fig. 27 is a graph showing the storage and loss modulus cross over points of the different HDPE carrier materials as function of angular frequency in a melt rheology frequency sweep,
Fig. 28 is a graph showing the delta value of the different HDPE carrier materials as function of angular frequency in melt rheology frequency sweeps,
Fig. 29 is an illustration of a typical unimodal (dashed) and two different bimodal molecular weight distributions (MWDs),
Fig. 30 are illustrations of examples of plastic carrier media from above, from the side and in three dimensions having a protected surface area utilized for biofilm formation in MBBR applications for treatment of contaminants in liquid streams,
Fig. 31 are illustrations of examples of plastic carrier media from above, from the side and in three dimensions having a protected surface area utilized for biofilm formation in MBBR applications for treatment of contaminants in liquid streams,
Fig. 32 are illustrations of examples of plastic carrier media from above, from the side and in three dimensions having a protected surface area utilized for biofilm formation in MBBR applications for treatment of contaminants in liquid streams
Fig. 33 are illustrations of examples of plastic carrier media from above, from the side and in three dimensions having a protected surface area utilized for biofilm formation in MBBR applications for treatment of contaminants in liquid streams,
Fig. 34 are illustrations of examples of plastic carrier media from above, from the side and in three dimensions having a protected surface area utilized for biofilm formation in MBBR applications for treatment of contaminants in liquid streams,
Fig. 35 are illustrations of examples of plastic carrier media from above, from the side and in three dimensions having a protected surface area utilized for biofilm formation in MBBR applications for treatment of contaminants in liquid streams,
Fig. 36 are illustrations of examples of plastic carrier media from above, from the side and in three dimensions having a protected surface area utilized for biofilm formation in MBBR applications for treatment of contaminants in liquid streams,
Fig. 37 is a picture of an example of a smooth inner wall inside a MBBR bioreactor,
Fig. 38 is a picture of an example of an inner wall with wall cavities inside a MBBR bioreactor,
Fig. 39 is a picture of an example of an inner wall with sharp objects inside a MBBR bioreactor,
Fig. 40 is a picture of an example of an inner wall with rough joints inside a MBBR bioreactor,
Fig. 41 is a picture of an example of an inner wall with non-smoothed joints inside a MBBR bioreactor,
Fig. 42 is a picture of an example of an inner wall with imperfect grades inside a MBBR bioreactor,
Fig. 43 is a picture of an example of an inner wall inside a MBBR bioreactor having degraded from smooth to rough over long time periods.

### Description of embodiments

The following description focuses on an embodiment of the present invention applicable to a plastic carrier according to the present invention with significantly increased resistance to withstand the rough conditions generally employed in different wastewater treatment plants, thereby significantly extending the carrier lifetime.

As such, not only is carrier lifetime extended for general MBBR applications, but the increased resistance may facilitate delay or eliminate refurbishment of older or worn equipment.

It may also facilitate better use of submersible mixers.

Similarly, it may facilitate utilization of higher than general mixing energies if necessary.

Furthermore, it may facilitate treatment of wastewaters comprising more severe plastic detrimental substances.

It may also facilitate utilization of MBBR technology as part of the treatment line for production of drinking water since the improved properties of the carrier media according to the present invention would be able to handle the rougher conditions for a much extended time and significantly reduce or eliminate the production of microplastics coming from the plastic carrier media compared to state of the art plastic carrier media.

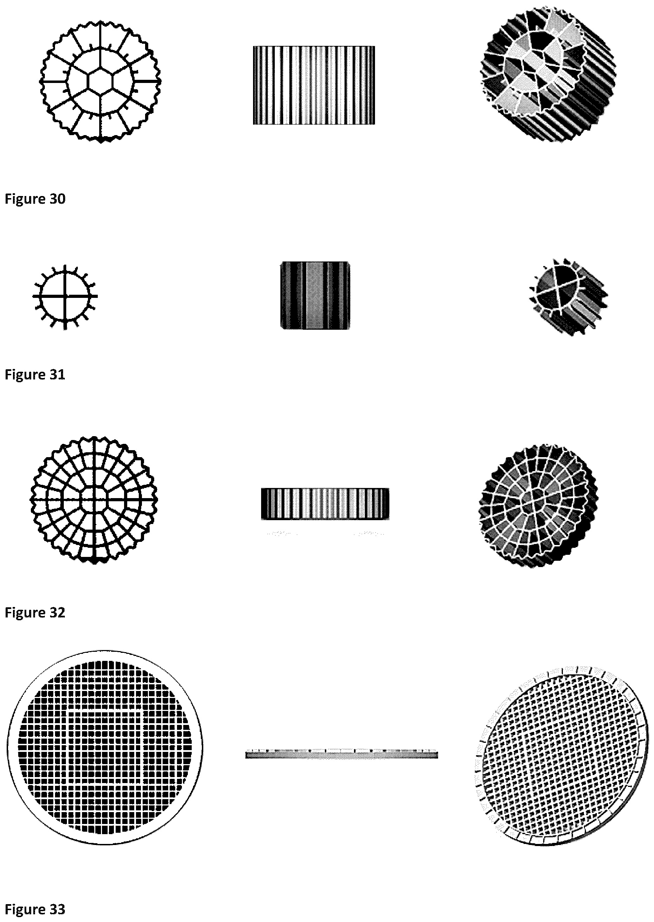

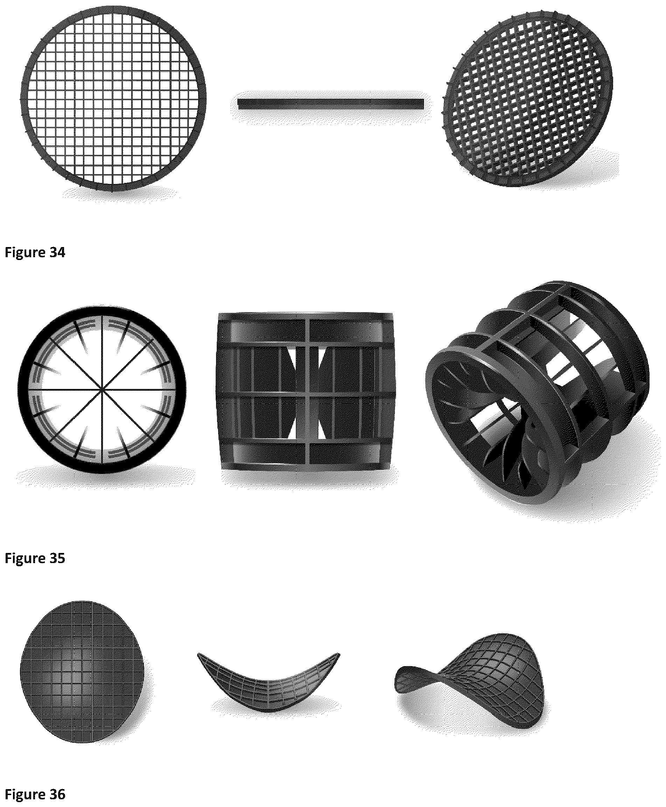

MBBR carriers are generally designed with a structure that allows for high surface area to accommodate biofilm growth (see examples of carrier designs in Figures 30 to 36). Usually, the carriers are produced via extrusion or injection moulding from one or more plastic raw materials which when molten or melt blended in the extruding processing equipment become the carrier material which in the melt stage is then formed into desired carrier design and shape before cooling the melt of carrier material into solid carrier pieces. However, carriers may be coloured by addition of colorant in the extrusion process, co-extruded with material inserts, such as metal strips for added functionality, various inorganics could be added in the extrusion melt process for changing density, whereby these will be part of the carrier material as well.

The carrier production process is generally continuous and carrier pieces can be formed individually in for example the case of injection moulding or by cutting cooled and solid long strands into individual pieces as in the case of extrusion.

MBBR carriers thus essentially consist of carrier material.

Plastic carriers for MBBR are generally made from polymer plastics.

One group of polymer plastics is plastic Polyolefins, which are high molecular weight hydrocarbons that include high-density polyethylene, low-density polyethylene, polypropylene copolymer, polymethyl pentene and polypropylene.

These represent the only plastics with a lower specific gravity than water, or in other words, they are the only common plastics that weigh less than water. Since the specific gravity of the plastic media for MBBR should be less than water in order to easily keep them flowing suspended in the water in the reactor volume without having to use too much energy, polyolefins is a good choice for plastic carrier media that is not foamed (foaming would naturally decrease the specific gravity of the material).

Out of the polyolefins the choice for production of carrier media for MBBR is polyethylene since it is a commodity polymer with very versatile and suitable properties for this type of application and it is easily shaped at high speed to the wanted design of the carrier media. Polyethylene can also be made to have to have a higher density (still below the density of water) than for example polypropylene and is thereby easier to keep in suspension because of lower buoyancy so that carriers do not just float around on the surface.

### General description of polyethylene

Polyethylene, like many other types of polymers, has a diverse material behavior with varying properties dependent on its structure and different types of polyethylene are readily commercially available. Its diversity can be explained by its molecular structure.

Polyethylene is a thermoplastic material composed of carbon and hydrogen atoms joined together forming high molecular weight products. Generally stated, ethylene with the application of heat and pressure is turned into polyethylene. The polymer chains may be hundreds of thousands to several millions carbon units (i.e. methylene groups) long. Short and/or long side chain molecules (branches) exist with the polymer's long main chain molecules. The longer the main chain is, the greater the number of atoms, and consequently, the greater the molecular weight. The molecular weight, the molecular weight distribution and the amount of branching determine many of the physical properties of the end product.

Molecular weight, its distribution and crystallinity (density) have the biggest impact on the properties of PE. The crystallinity in turn depends on molecular weight and degree of branching. The less the polymer chains are branched, and the lower the molecular weight, the higher the crystallinity of polyethylene.

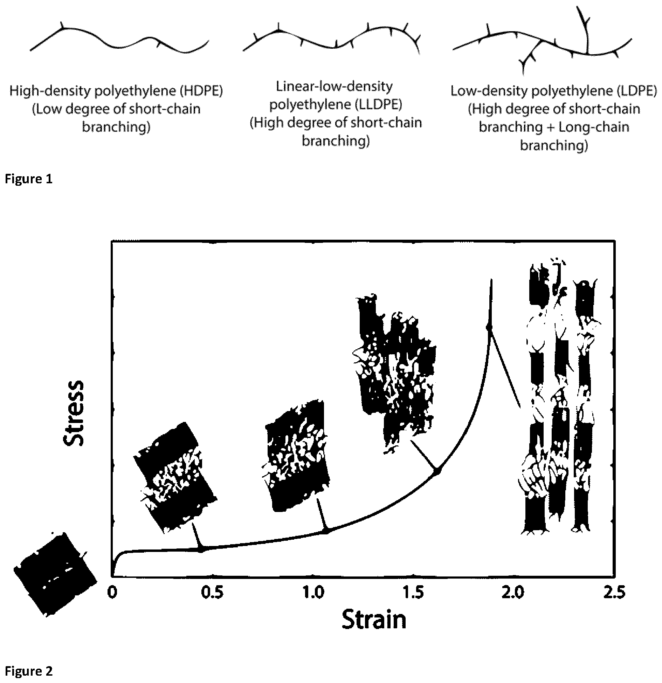

The most common types of polyethylene are low density polyethylene (LDPE), linear low density polyethylene (LLDPE) and high density polyethylene (HDPE). LDPE has a high degree of short and long chain branching along is backbone chains, while LLDPE has a high degree of only short-chain branching and HDPE has a low amount of short-chain branching (Fig. 1). The LDPE is therefore more restrained (because the molecule is less linear) to form crystalline regions upon cooling from melt lowering the density because the polymer chains cannot fold as easily into a highly ordered crystalline folded structure. The LLDPE and HDPE on the other hand have more linear polymer chains (especially HDPE) increasing their crystallinity and thereby giving them superior mechanical properties and higher stiffness needed for an application as MBBR. LLDPE however has a lower density (0.91-0.93 g/cm³) than HDPE (0.93-0.97 g/cm³) due to a larger amount of branching making it less suitable than HDPE for use as material for production of carrier media to be used in MBBRs.

According to an embodiment, HDPE, as used herein, relates to polyethylene having a density of 0.93-0.97 g/cm³. Typically, its density exceeds 0.93 g/cm³.

According to an embodiment, HDPE, as used herein relates to either a homo-polymer or a co-polymer, wherein the homo-polymer is produced from the monomer ethylene, while the co-polymer additionally comprises at least one other monomer alkene, such as 1-butene, 1-hexene or 1-octene.

### General routes of failure of MBBR plastic carriers

Plastic carriers for MBBR (examples of, but not limited to, different types of carrier media are seen in Figs. 30-36) applications need to withstand harsh environments during extended periods of time. When used in MBBR plants world-wide for wastewater treatment they need to withstand among other things concentrated and constant shear forces from mechanical mixers, constant shear forces from wall impacts, constant shear forces from carrier-carrier impacts, seasonal temperature changes and all kinds of chemical substances both present in the wastewater and used in the wastewater treatment process for extended time periods in an aqueous media. The environment could become even harsher if the reactor wall conditions are poor (for example torn and rough walls) or for utilization of high speed mixers, high aeration rates (thereby higher shear forces and more collisions) or a high degree of carrier filling compared to reactor volume (thereby more collisions). These environments with time will ultimately make the plastic carrier pieces break and reach their end lifetime. Naturally every WWTP would like an as long carrier media lifetime as possible since it comes at cost to replace them with new plastic carrier pieces and replacing existing carriers would temporarily disrupt the biological treatment process since new biofilm would have to be grown on the new carrier media. Additionally, if carriers are not replaced in time before breakage, broken media pieces may go through the sieves and ultimately leave the WWTP and be spread into nature.

Wastewater treatment plants employing MBBR technology sooner or later have to exchange plastic carrier media when it has reached its end lifetime. The lifetime of the employed carrier media may vary widely in light of the different environmental conditions at different wastewater treatment plants as described above and if the conditions are rougher the lifetime will be shorter but could vary between, but not limited to, 5-25 years dependent on the conditions. However, the type of polymer material used for production of plastic carrier media may also play a large role in determining the carrier lifetime. Naturally different types of materials have different properties and some of these different properties may be of great importance for improving the lifetime of carrier media utilized in an MBBR process. If one could identify the property or properties for in this case HDPE (which is chosen in the invention for its properties as raw material for carrier media production as explained before) which affect the carrier lifetime in an MBBR process the most one could also make an effort to try and modify said HDPE material and thereby improve that specific property or properties without impairing the other properties of the HDPE needed to have a functional MBBR process.

To identify the most important property for the raw material to have to produce plastic carrier media with extended lifetime one first have to understand why and how carrier breakage occurs. Carrier breakage can be caused for numerous reasons but can generally be divided into three categories:
- Molecular degradation of the HDPE material structure due to for example UV degradation or chemical substances attack on the polymer backbone chains resulting in chain scission and deterioration of physical properties which in combination with continuous shear stress (high or low) in the reactor break the carriers. If the molecular structure is unchanged that would mean that mechanical properties could be restored if the material was molten and shaped again into a carrier. If the molecular structure is degraded the material would forever have lost its mechanical properties even if re-molten.
- Physical degradation over time of the HDPE material due to environmental factors such as continuous shear stresses, temperature changes and chemicals causing stress cracking in the material. This is different to molecular degradation as it does not break polymer bonds in the main polymer backbone chains but instead breaks the secondary linkages, e.g. van der Waal bonds, between polymer chains. These are broken when the low mechanical stresses cause stretching and eventual chain slippage over time of the amorphous phase of the polymer causing cracks to propagate and eventually break the plastic. Stress cracking is accelerated by for example higher temperatures, increased stress concentrations and chemical concentrations and ultimately leads to brittle failure. Stress cracking can be withheld for significantly longer time if the shear stresses in the system are kept to minimum possible. Physical degradation can be reversed by re-melting the material but of course in terms of MBBR carriers this is not practically feasible.
- Plastic deformation - Too high shear forces in the MBBR reactor being higher than the material yield stress as virgin material leads to the material starting to yield and stretch until sudden ductile failure which could be a way of failure of MBBR carriers if sudden significantly high shear forces a applied in the reactor, for example if carrier media gets stuck in mechanical mixers or elsewhere where significant force is applied to the stuck carrier.

If the molecular degradation of the plastic material can be excluded (this can be measured) it would be either the plastic deformation or the physical degradation of the plastic over time that caused the carrier media to eventually fail and reach is end of life.

The physical degradation over time and the plastic deformation, respectively, lead to two types of mechanical failures for plastic materials. Ductile fracture (from plastic deformation) and brittle fracture (from physical degradation over time). Ductile failure is a type of failure that generally occurs over a short amount of time at high stress levels. At the macroscopic level, ductile tensile failure results in observation of visible deformation (necking) in the polymer sample. Compared to ductile failure, a polymer suffering brittle failure has a clean break with little material deformation due to being stressed by lower stress levels for an extended period of time.

Tensile ductile behavior is influenced by the semi-crystalline nature of the material. In Fig. 2, the stress-strain curve for tensile ductile deformation is accompanied by illustrations of what occurs within the semi-crystalline polymer matrix at the micro scale. At the beginning, before the yield point, no visible deformation of material is observed and the load is mainly carried by rigid crystalline lamellae. As strain increases, stress increases as well and yield occurs. During the time period between the point of yield and the onset of strain hardening, the load on the test sample remains at a relatively constant level. The deformation in this region is due to a combination of amorphous phase rearranging itself and crystal lamellae slipping past each other, however each individual crystal itself is still intact.

Between strain values of 0.5 and 1.0 in Fig. 2 increasing orientation of the crystalline and the amorphous phases in the direction of drawing is seen with increasing stress-strain values. After the strain of 1.5, sharp increase in stress value with increasing in strain indicates the occurrence of strain hardening. During strain hardening, the amorphous phase has reached its full extension, and further deformation of the polymer in this stage is due to breaking and unfolding of crystalline lamellae. The breaking of crystalline lamellae into smaller chunks results in the characteristic rough fibrous surface of ductile failure, as can be observed under a scanning electron microscope (SEM). As stress continues to increase with increasing strain, ultimate failure occurs and the material breaks.

In comparison with ductile failure, a polymer that undergoes brittle failure has a clean break with little material deformation. To the eye, the fracture surface appears smooth. Under an SEM, it can be seen that the surface actually consists of short random pull-outs. Brittle-type failure occurs when a low stress is applied over a long period of time.

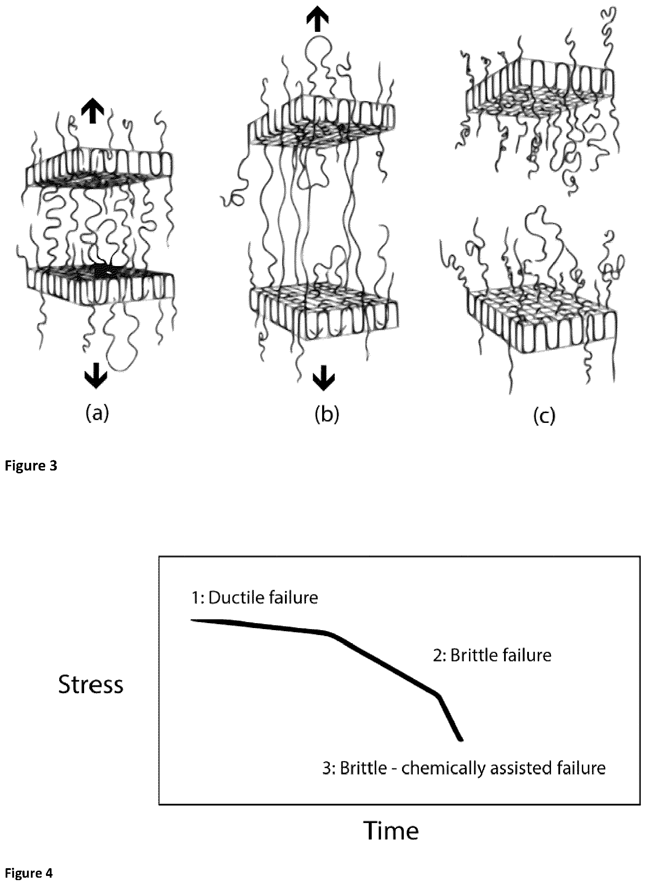

As shown in Fig. 3 a-b, in the initial steps of brittle fracture, the amorphous phase starts to stretch under stress. Due to the longer time period, the inter-lamellar links in the amorphous phase under stress start to relax and untangle from each other until the number of remaining linkages becomes very low. When the few remaining inter-lamellar links are stretched to their limit, they are unable to pull apart crystalline lamellae, consequently, a brittle fracture of the polymer occurs, as illustrated in Fig. 3 c.

Stress Crack Growth (SCG) is a phenomenon in semi-crystalline materials, such as HDPE, whereby slow growing cracks can occur due to the presence of stress in the material. The long term durability of the material can be dependent upon its resistance to inhibit the initiation and slow growth of cracks. Research has shown that SCG is one of the three major failing modes (Fig. 4) for semi-crystalline polymers.

Ductile failure mode I results in yielding and reflects a material's propensity to undergo large-scale, irreversible 'plastic' deformation when under stress. The mechanism results in localized expansion and final rupture of the deformed zone.

Failure Mode II is associated with creep, creep rupture and SCG. Creep is a time-dependent, non-reversible deformation, when exposed to a constant tensile stress. Creep rupture is the terminal event of creep and is a measure of the time that a material under a constant, applied tensile load takes to fail.

Creep rupture can be accelerated by;
- Temperature
- Stress concentrations
- Fatigue
- Chemical environment

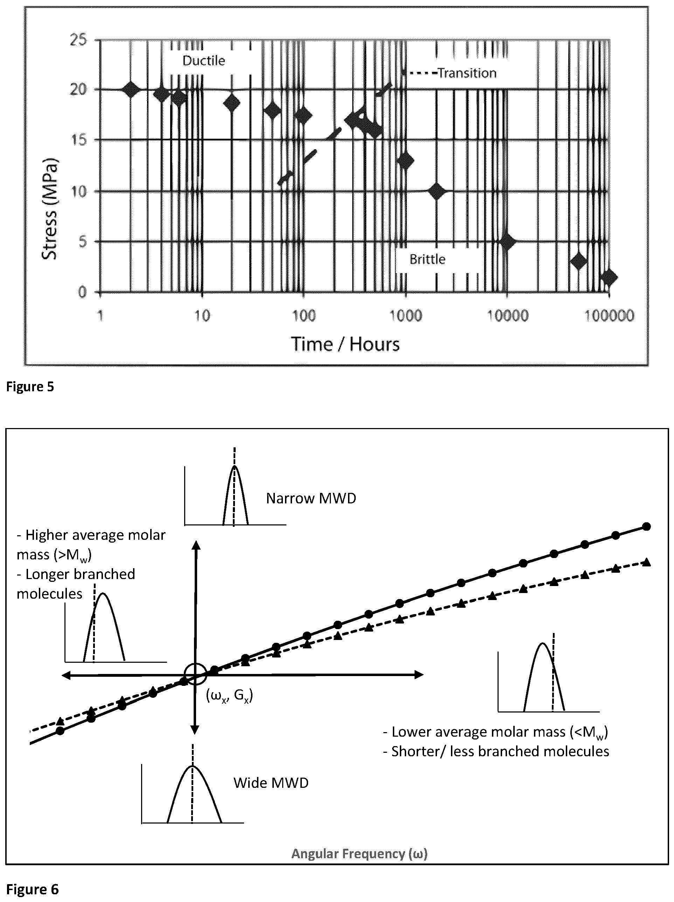

Fig. 5 is representative of a creep rupture curve where the ductile-brittle transition signals the onset SCG

After crack initiation voids develop ahead of the crack. These voids gradually merge into larger voids that are spanned by highly orientated load bearing fibrils. This process known as crazing continues until a point is reached when the most highly stretched fibrils will rupture resulting in fracture.

Now it is of importance to investigate MBBR carrier media that has been in operation for a significant time and has reached or is very close to its end of life. By doing so it would be possible to determine which failure route that has caused its end of life. Thereafter it would be possible to determine what could be done to the plastic raw material in order to cut off that failure route as well as possible.

The first thing to investigate would therefore be to confirm whether molecular degradation of the plastic raw material had taken place in two different MBBR processes having two different types of carrier design (in terms of size, form and shape) and had operated long enough for the carrier media in both plants to reach end of life. This was done by comparing the molecular structure of the HDPE raw material in virgin unused carrier media, which was the same for both types of media, to the molecular structure of the raw material in the two different carrier media having reached end of life.

In order to compare the molecular structures of virgin HDPE raw material and HDPE in the carrier media having reached end of life melt rheology measurements of the different materials were performed. Thermoplastic polymers like HDPE are viscoelastic fluids when heated above their melting temperature. That means they behave viscous or elastic in the melt state, depending on how fast they flow or are deformed in its melt processing.

The polymer structure-rheology relationship is a key to the development of polymers with the correct rheology for processing. The ultimate goal of the design engineer is to modify the material structure to give better melt processing performance without sacrificing the performance of the final product (for example mechanical properties). Due to its sensitivity to polymer structure changes, melt rheology is the desired technique to very accurately compare molecular structure differences between different HDPE raw materials. The transformation process reacts very sensitively to small variations of the material, i.e. the rheology of the polymer melt is very sensitive to small changes of the polymer structure.

### Determining the route of failure for MBBR plastic carriers

Because of the sensitivity of rheology, it is a most convenient method to characterize polymers. A small amount of a high molecular weight polymer can change the processing behavior dramatically and so does the melt rheology. The important structure parameters defining the rheology of the polymer melts are molecular weight (MW), molecular weight distribution (MWD) and branching. Whereas an increase of the molecular weight causes the viscosity to increase, changing of the molecular weight distribution and branching mainly affects the elasticity of the melt. The time dependence is affected by both.

Rheology measurements will therefore provide exact and reliable data to determine whether the specific HDPE raw material used to produce the carriers having reached end of life has molecularly degraded or not during its employment in a specific MBBR process.

The loss modulus characterizes the material's viscous properties i.e. the energy dissipation. The storage modulus is a measure of the elastic properties and specifies the energy stored in the material. In the range of the Newtonian plateau, the loss modulus curve has a constant slope at low angular frequencies and the storage modulus has another constant slope at low frequencies when the data is presented in a double logarithmic scale. In this region the loss modulus G" is running above the storage modulus G' meaning the sample behavior is similar to that of a fluid. By increasing the angular frequencies, the so called cross-over point (COP) between storage modulus and loss modulus indicates a transition from a more viscous like deformation behavior to more elastic deformation behavior. The COP can therefore be used as a criterion for a qualitative material characterization.

Information one get from the position of the COP is qualitative information about the material's average molar mass and its molar mass distribution (MMD). An increasing average molar mass is expressed in a COP moved to lower angular frequencies. At higher angular frequencies shorter molecules remain mobile, whereas longer molecules become immobile already at lower angular frequencies. A vertical shift of the COP towards lower modulus indicates a wider MMD. This can be seen in Fig. 6. At angular frequencies above the COP there is not enough time left for the molecules to disentangle. To a given strain or shear rate the material has a response like rubber. Elastic properties are dominating over the viscous or fluid properties. The region is the so called rubbery region.

Since a frequency sweep as according to Fig. 6 gives detailed information regarding the molecular weight, molecular weight distribution and branching two different end of life carriers taken from two different MBBR reactors were compared to the virgin HDPE material before utilized in the plants and analyzed to determine their respective Gₓ and ωₓ at the cross over point as according to Fig. 6. The aim was to establish these values for each sample in order to determine if they have changed or not. If not the molecular structure of the end of life HDPE samples would be intact and would in such case not be the cause of the MBBR carrier failure.

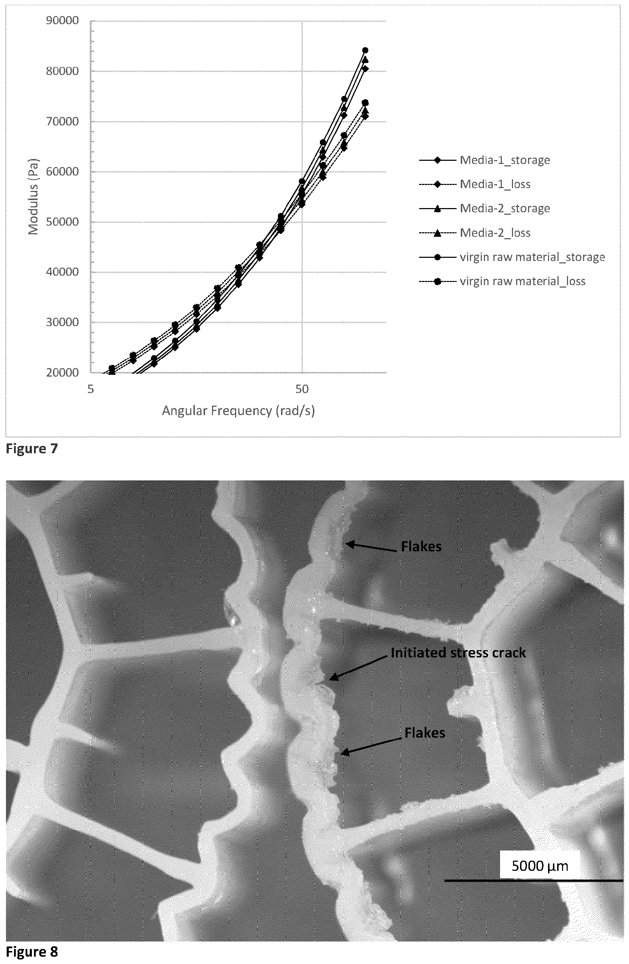

Fig. 7 shows the comparison of two different end of life carriers (Media-1 as depicted in Fig. 30 and Media-2 as depicted in Fig. 31) after use in two different MBBR processes and the virgin HDPE material as before use. It is seen in Fig. 7 that the frequency sweep melt curves basically lie on top of each other with very small differences. The cross over point values for all analyzed samples can be seen in Table 1. These results clearly show that there has been no molecular degradation of the HDPE raw material during the operation in the MBBR process meaning the carrier failure and end of life must be due to physical degradation, either through physical degradation over time (formation of stress cracks) or through plastic deformation.

**Table 1: Values of crossover points (COPs) coordinates for end of life Media-1, end of life Media-2, and virgin media before use**

| Property | Media-1 | Media-2 | Virgin media |
|---|---|---|---|
| G_{X} (Pa) | 46470 | 47010 | 47060 |
| ω_{X} (rad/s) | 36.5 | 35.7 | 34.2 |

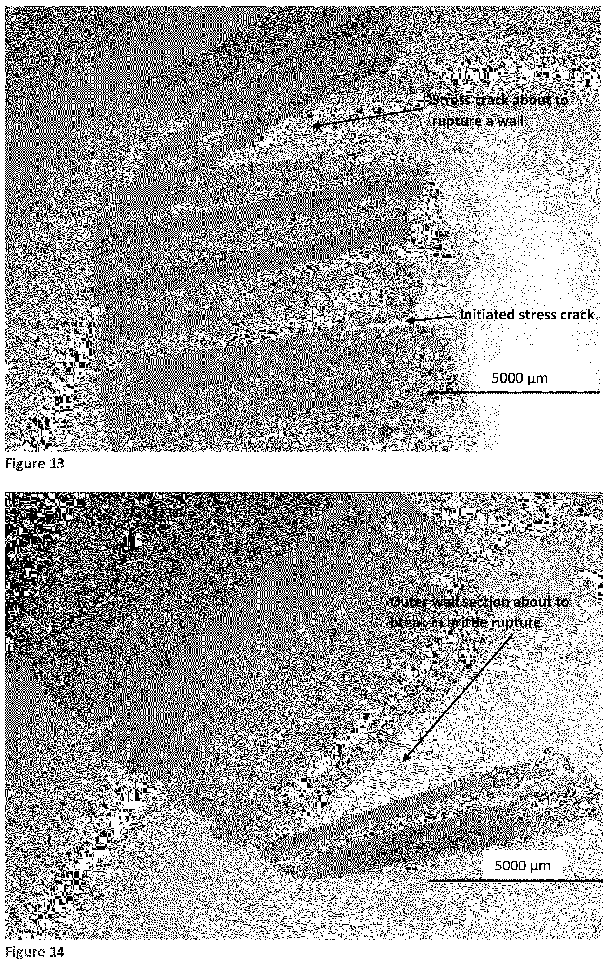

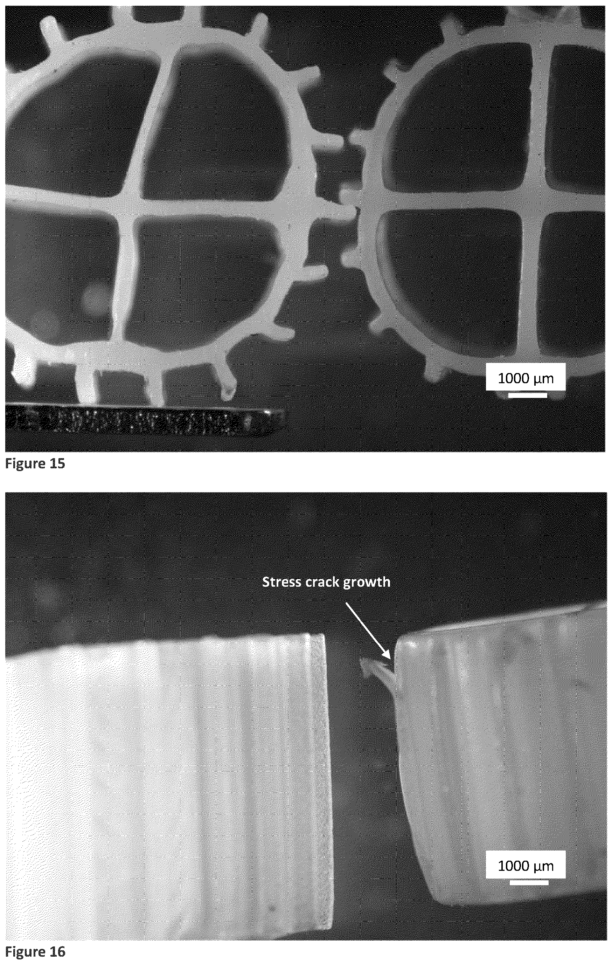

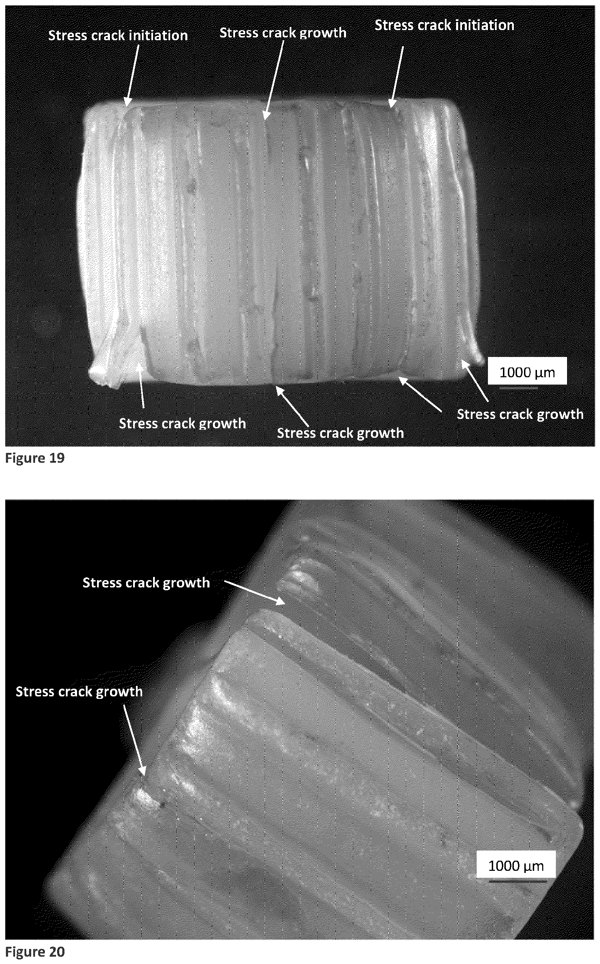

Since no molecular degradation had taken place the physical degradation route had to be investigated. This was done utilizing light microscopy. Microscopic pictures of two different types of broken carrier media piece samples from two different MBBR processes are shown in Figures 8-14 for process number one (Media-1) and in Figures 15-21 for process number two (Media-2).

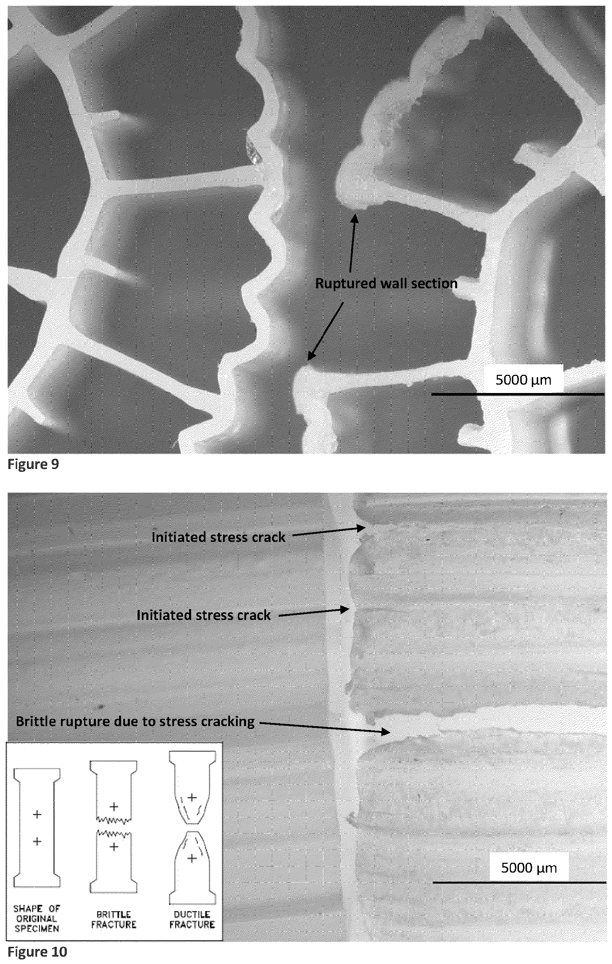

Figures 8 and 9 show virgin media to the left, a non-broken part of a carrier (Media-1, Fig. 8) and a broken part of a carrier (Media-1, Fig. 9) to the right. From these images it is evident that the wall thickness has not been particularly affected during the time of operation of the MBBR reactor. Therefore, not much scraping/erosion of walls were seen. This was confirmed in all of the pieces tested (but not shown here), also the broken ones. That meant that media erosion from scraping by wall thinning has little or nothing to do with the media breakage. However, in Fig. 9 it can be seen to the right that an outer wall part of the carrier has been cut out in a clean break by the perpendicular inner walls at each side.

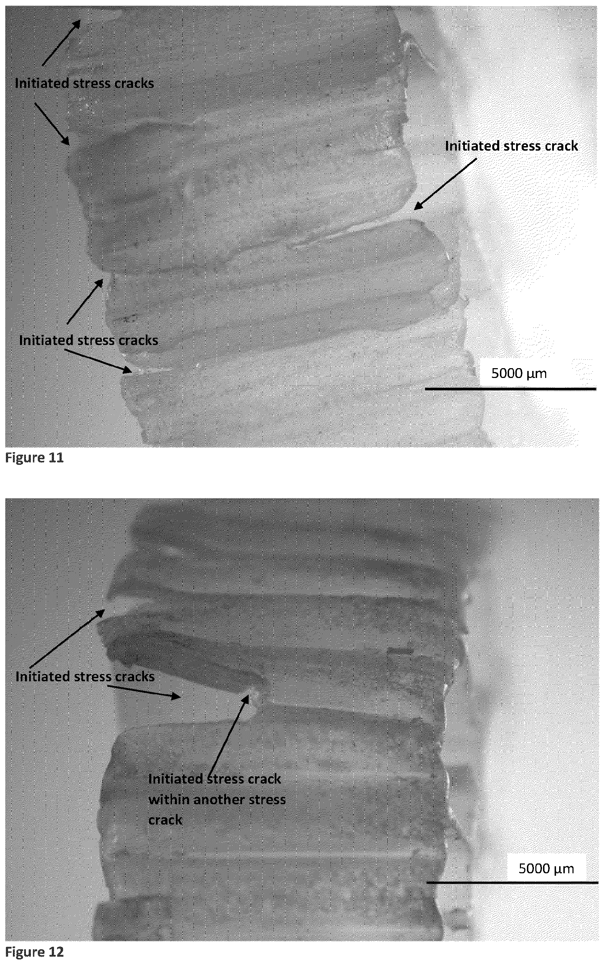

Fig. 10 shows virgin carrier media on the left and a damaged carrier media piece (Media-1) standing on its side. Figures 11-14 show damaged carrier media (Media-1) standing on its side. From the stand up angle formed stress cracks are easily seen in all figures 10-14. Both smaller initiated cracks that have just started to grow, larger stress cracks which have grown larger and are on the brink of fracturing the outer walls caused by the stress cracks which always end with brittle fracture as depicted in Fig. 10.

Fig. 15 shows virgin media to the left and a damaged carrier media (Media-2 having another design shape compared to Media-1) from a different MBBR process. From this image it is evident that the wall thickness has not been particularly affected during the time of operation of the MBBR reactor as was also the case for Media-1. Therefore not much scraping/erosion of walls were seen. This was confirmed in all of the pieces tested (but not shown here). That meant that media erosion from scraping by wall thinning has little or nothing to do with the media breakage. It can be seen in Fig. 15 that the outer fins are shorter which may be due to scraping/erosion however Fig. 16 points out that this is due to another cause.

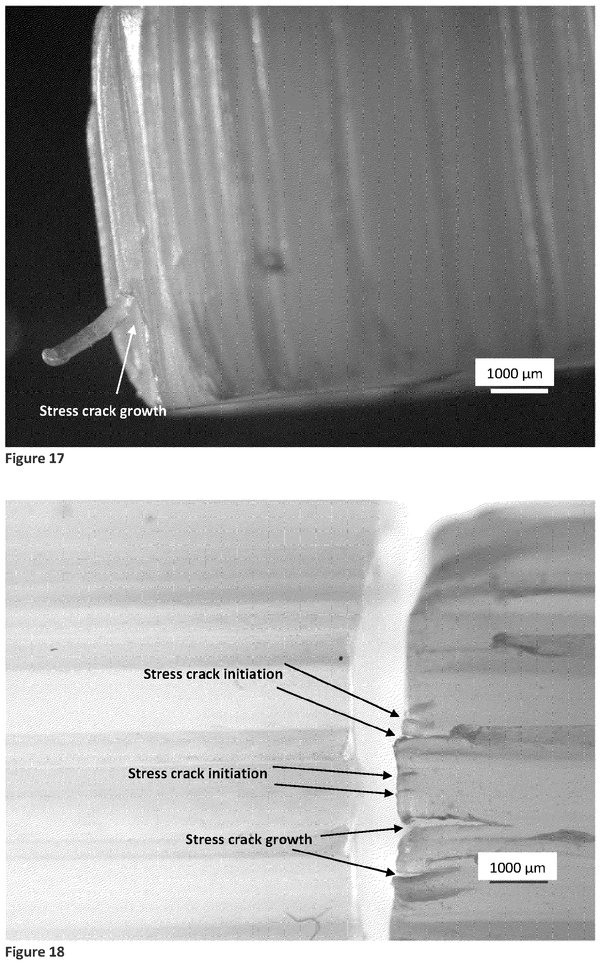

In Fig. 16 it can be seen that a stress crack has been initiated and started to grow in the vertical direction of the outer fin of Media 2 beginning to separate the fin from the outer wall. This stress crack propagation ending with brittle fracture and with the fin falling off the outer wall is likely why the fins are shorter in Fig. 15 compared to the virgin media. Not due to erosion. This is also more clearly seen in Fig. 17, where the stress crack is growing along the vertical axis of the fin in the notch of the outer wall. This is clear behavior of stress crack initiation, propagation and eventual rupture due to continuous low stress endurance during a long period of time.

Typically, the outer parts of the carrier (outer walls in the case of Media-1 and outer fins and walls in the case of Media-2) takes up most of the shear stress in the reactor and when they have been partly or fully scaled off the continued shear stress will be applied in the 90 degree corner of the outer walls. At that point the stress cracks will instead be initiated and grown from there in the outer walls as seen in Figures 18-20.

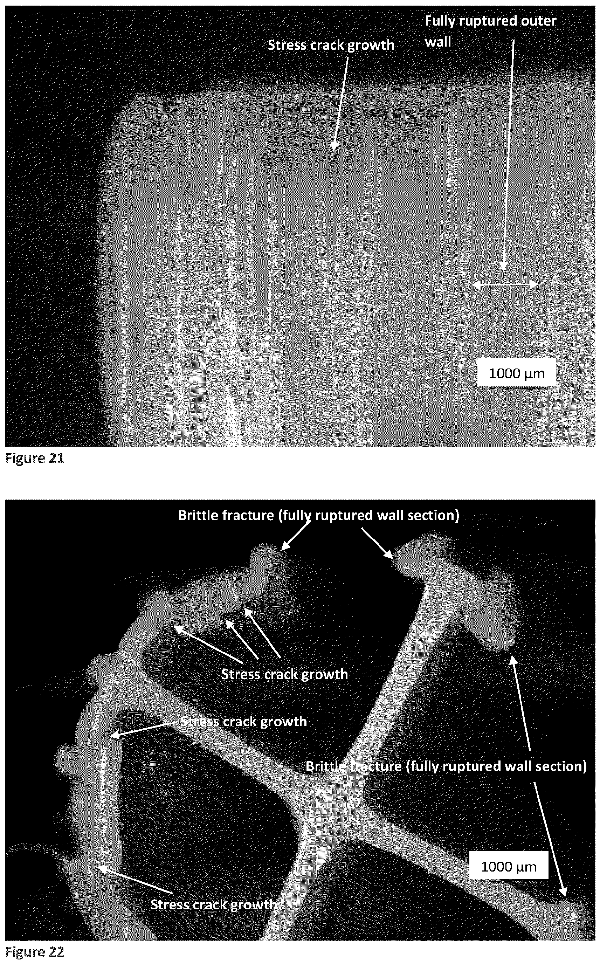

Finally, the outer wall ruptures as seen in Fig. 21 where a full brittle rupture can be seen at one specific place in the outer wall. Naturally cracks are initiated at different times and propagate at different rates but eventually two cracks have propagated enough to lead to fracture and thereby sever a full section of an outer wall as seen in Fig. 22. Generally, the first ruptures occur in the corners between inner and outer walls because of more shear stress being absorbed by this "heavier" area. When all outer wall sections have finally been severed the shear stress in the reactor will be taken up by the inner walls which will then start to deteriorate in the same way.

It was therefore surprisingly found that the failure mechanism for the carrier media in the MBBR process was brittle rupture due to stress crack formation and propagation. This was not expected, since the tested Media-1 and Media-2 were different carrier types, which have been used in very different processes for different types of waste waters. While Media-1 had been used in an aerobic process using, media-2 had been used in an anoxic process using submerged mechanical mixing, why one would expect different routes carrier failure for media-1 and media-2.

It therefore became evident that the HDPE raw material structure utilized for production of MBBR carrier media should be enhanced to have better properties in terms of resistance to stress crack formation.

### Ways of Increasing the lifetime of MBBR plastic carriers

The tenacity of the polymer and its resistance to brittle rupture will be highly dependent on molecular architecture, particularly molecular weight, molecular weight distribution, branching, crystallinity and tie molecules. The tie molecules are embedded in the crystallites and transverse amorphous regions (see Fig. 3) act as mechanical links between the crystalline domains and therefore play a decisive role in the resistance to brittle failure and overall mechanical properties when subjected to stress.

It was explained previously that brittle fracture is believed to be caused by disentanglement of inter-lamellar links. The number and type of these tie-molecules play an important role on the stress cracking resistance of polyethylene. There are two types of inter-lamellar linkages.

The first kind is what is called bridging tie-molecules. The two ends of these molecules are embedded in two different crystalline lamellae, thus connecting them. Bridging tie-molecules have strength due to covalent bonds. The other type of inter-lamellar link is made of entanglements of loose loops and cilia and is believed to be held together by van der Waals forces. Bridging tie-molecules will be referred to as tie-molecules. All other types of inter-lamellar linkages will be referred to as entanglements.

The concept of tie-molecules was first proposed by Brown and Ward in their study of brittle fracture of polyethylene. Brown and Ward theorized that there are two types of load-bearing molecular bonds in the amorphous phase of polyethylene. The first type consists of the covalent bonds of bridging tie-molecules, whereas the second type involves van der Waals bonds between amorphous chains. The brittle fracture stress σ_{F}, of a polymer is therefore a sum of the stresses carried by both type of bonds

Based on the work by Brown and Ward, Huang and Brown theorized that a polymer chain must have an end-to-end distance (r, radius of gyration) larger than the thickness of two crystalline lamella layers in order to crystallize in two lamellae and hence become a tie-molecule (see again Fig. 2).

Huang and Brown considered that there are only three types of amorphous phase materials, namely cilia, loose loop, and tie-molecules and any chain with end-to-end distance greater than 2L has an equal chance of taking on any one of the three amorphous phase configurations. Therefore, a chain only has 1/3 chance of becoming a tie-molecule.

The radius of gyration of a molecule is a function of its molecular weight. Based on probability theory and (empirical) experimental observations, Huang and Brown developed theories to account for the fraction of the area of the amorphous region occupied by bridging tie-molecules as a function of weight-average molecular weight (Mw) of the polymer.

The work showed that below a certain molecular weight no tie-molecules could be found. Other research also found that as weight-average molecular weight increases, the number of tie-molecules formed also increases. This means that the stress cracking resistance of polyethylene increases as weight average molecular weight increases, since the tie-molecule concentration increases.

The Huang and Brown model gave a good explanation for molecular weight effects on stress cracking resistance of polyethylene. In the last twenty years, most studies on stress cracking resistance of polyethylene have thus focused solely on the effect of tie-molecules. However, Huang and Brown's theory could not account for the higher stress cracking resistance of polyethylene with higher co-monomer (small inclusions of for example 1-butene or 1- hexane into the polyethylene backbone) content in the high molecular weight end of the molecular weight distribution. In addition, even though van der Waals bonds are much weaker than covalent bonds, Brown and Ward felt they should not be ignored. Other research in the last ten to twenty years also speculated that in addition to tie-molecules, other inter-lamellar links (i.e. chain entanglements) could contribute to the overall environmental stress cracking resistance of polyethylene.

Short Chain Branching (SCB) affects polymer properties by encouraging chain entanglements and at the same time reducing material density. Research has shown that when SCB content increases from 0 to 4.6 butyls/1000 carbon atoms, the observed rate of slow crack growth decreases by a factor of 104. Janimak and Stevens demonstrated the relationship between short chain branching and tie-molecule density. They charted their results with data from Huang and Brown on a plot of tie-molecule fraction versus branch density. Both sets of data showed an increase in tie-molecule fraction with an increase in SCB. In addition to the number of SCB, the length of the SCB also has an effect on the stress cracking resistance of polyethylene. Work done by Yeh et al. found that the stress cracking resistance of polyethylene increased dramatically as SCB length increased from 2 to 6 carbon atoms. The reason for this is believed to be the increasing sliding resistance of the chain with longer SCB branches.

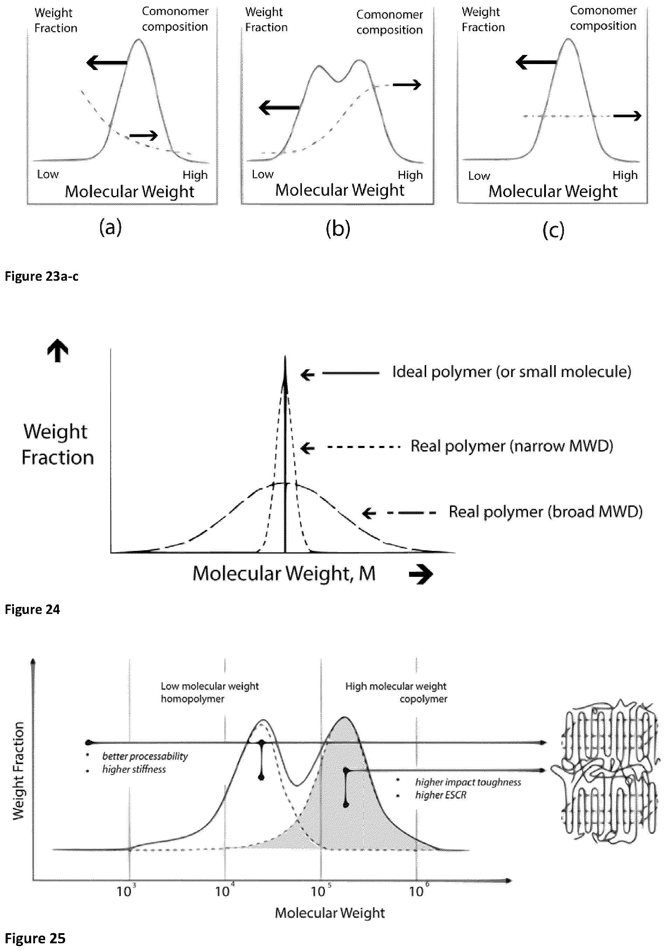

The type of catalyst used in polymerization affects short chain branch distribution (SCBD) in polyethylene. Polyethylene produced using Ziegler-Natta catalysts is known to have higher SCB content in the low molecular weight end of the molecular weight distribution (MWD), as shown in Fig. 23a. Use of Ziegler-Natta catalysts in tandem polymerization reactors on the other hand can produce PE with more short chain branches in the high molecular weight end of the MWD (Fig. 22b), while these polyethylene materials also tend to have a bimodal MWD. Investigations on metallocene catalyst have shown that short chain branches are evenly distributed across the MWD as illustrated in Fig. 23c. For metallocene catalyzed PE and tandem polymerized PE, presence of SCB in higher MW chains results in greater tie-molecule density and thus in a greater disruption of the regular chain folding mechanism for lamellae formation. Hence, these two types of polyethylene would in general have higher stress cracking resistance than polyethylene produced using Ziegler-Natta catalysts in the standard process.

SCB facilitates the formation of tie-molecules. However, SCB also disrupts the regularities of the crystallite and undermines the strength of the crystallite. Decrease in crystallinity means lower material density. Density is directly associated with the stiffness and tensile yield strength of polymer. By incorporating different amounts of model tie-molecules into linear polymers, it is found that an increase in tie-molecule density past a certain point results in loss in polymer crystallinity and tensile strength. For polyethylene used in applications such as MBBR wastewater treatment plants, both high environmental stress cracking resistance and high mechanical stiffness and strength are desirable qualities. It is therefore of importance to balance the two properties. Therefore, it was hypothesized that due to the nature of the damages found, utilizing HDPE with a bimodal production technology including chain branching in the high molecular weight end of the MWD (tandem process) would generate a high enough density for stiffness and tensile yield strength while also facilitating a much improved stress cracking resistance for use as raw material for the production of plastic carrier media for MBBR with a much prolonged lifetime.

As a summary the stress cracking resistance will be affected by the parameters shown in Table 2 in the way stated.

**Table 2: Dependence of the stress cracking resistance (SCR) on molecular weight (Mw), Molecular weight distribution (MWD), short chain branching (SCB), short chain branching distribution (SCBD) and long chain branching (LCB)**

| | **Property change** | **Effects** | **ESCR** |
|---|---|---|---|
| **MW** | ↑ | • Increases chain entanglements | ↑ |
| | | • Increases tie-molecule concentration | |
| | | • Increases lamella lateral surface area | |
| | | • Increases crystallinity and strength | |
| **MWD** | ↑ | • Improves processability | ↑ |
| | | • Increases chain entanglements | |
| **SCB** | ↑ | • Increases chain entanglements | ↑ |
| | | • Reduces chain slippage | |
| **SCBD** | Related to SCB | • Higher SCB content in the high MW end of MWD increases inter-lamellar link formations | ↑ |
| **LCB** | ↑ | • Increases chain entanglements | ↑ |
| | | • HDPE does not generally have sufficiently high levels of LCB to affect its ESCR | |

### Raw materials for improving the lifetime of MBBR plastic carriers

There are generally speaking three different main types of HDPE falling into different categories dependent on how they are produced. These categories are; unimodal homopolymers of HDPE, unimodal copolymers of HDPE and bimodal homopolymers or copolymers of HDPE.

The molecular structure of HDPE homopolymer is a linear backbone of the repeating unit (-CH2-CH2-), meaning only ethylene is used as monomer for production as it is a homopolymer produced from a single monomer type. This type of HDPE is produced using one catalyst in one reactor. The result of this process is a polymer with a reasonably broad molecular weight distribution (Broad MWD) as can be seen in Fig. 24. For example, let's state that a sample of polyethylene with some of its chains is having fifty thousand carbon atoms in them, and others having fifty thousand and two carbon atoms in them. This small difference isn't going to amount to anything. However, one almost never finds a sample of a synthetic polymer in which all the chains have the same molecular weight. Instead, usually a bell curve or a distribution of molecular weights can be seen. Some of the polymer chains will be much larger than all the others, at the high end of the curve. Some will be much smaller, at the low end of the curve. The largest number will usually be accumulated around a central point, the highest point on the curve. For a unimodal polyethylene homopolymer the broad MWD in Fig. 24 is given.

With the evolution of increasingly sophisticated polyethylene technology, alpha olefins 4, 6 and 8 have become valuable co-monomers for the production of a wide range of unimodal copolymer HDPE resins. That means not only ethylene is used as monomer for the HDPE production anymore but also small inclusions of for example 1-butene, 1-hexene or 1-octene can be incorporated into the polymer backbone chains. By including these monomers the chain branching can be increased.

In one embodiment, the HDPE comprises ethylene and at least one other alkene, such as 1-butene, 1-hexene or 1-octene, making it an HDPE co-polymer.

Unimodal homopolymers of HDPE possess high flexural stiffness (flexural modulus) but low stress cracking resistance (ESCR). Lowering the melt index (increasing the melt viscosity meaning increased average molecular weight) can help to alleviate the low stress cracking, but the processability of the polymer suffers, due to lower flow rates. The addition of alpha olefins 4, 6 or 8 as a co-monomer increases the resulting polymer short chain branching, thereby improving the flow properties while increasing the stress cracking resistance.

The emergence of new applications with even more severe operating requirements has pushed the performance demands on HDPE resins to new levels. Although today's class of unimodal HDPE resins generally perform very well in terms of physical properties and stress crack resistance (SCR), bimodal HDPE resins represent an even further significant improvement in all of these properties. Unimodal HDPE resins are produced using one catalyst in one reactor. The result of this process is a polymer with a reasonably broad molecular weight distribution as shown in Fig. 24. This broad range of polymer chain sizes includes both smaller molecules, which affect processability (e.g. extrusion flow rates), and much larger molecules, which influence physical properties such as mechanical properties and stress cracking resistance. Density is a critical attribute for PE resins. For a given polyethylene material, reducing density improves many important physical properties related to ductility (or lack of brittleness), such as SCR and many mechanical properties.

Density is controlled by the incorporation of co-monomers into the polymer at relatively small levels during polymerization. These create short side chain branches, which act to disrupt the crystalline structure and lower density. However, this process is not completely efficient, because the co-monomer preferentially goes into the smaller, lower molecular weight chains (see Fig. 23a), which are less effective (than the longer polymer chains) at influencing physical properties. This tendency for co-monomer incorporation into shorter polymer chains limits stress crack resistance and certain physical properties for a unimodal resin at a given density.

Bimodal resins are based on essentially the combination of two polymers, a high molecular weight (HMW) polymer and a low molecular weight (LMW) polymer.

Normally, these resins are produced using two polymerization reactors in series (LMW and HMW), each operated under separate process conditions. This process allows all of the co-monomer to be incorporated into the high molecular weight fraction, where it is most needed to influence properties (Fig. 25). The result of this technology is a substantial leap in physical properties at a given resin density. Ultimately, this range of improved performance may equal longer service life and increased confidence that the carrier will maintain its integrity through more demanding type of environments.

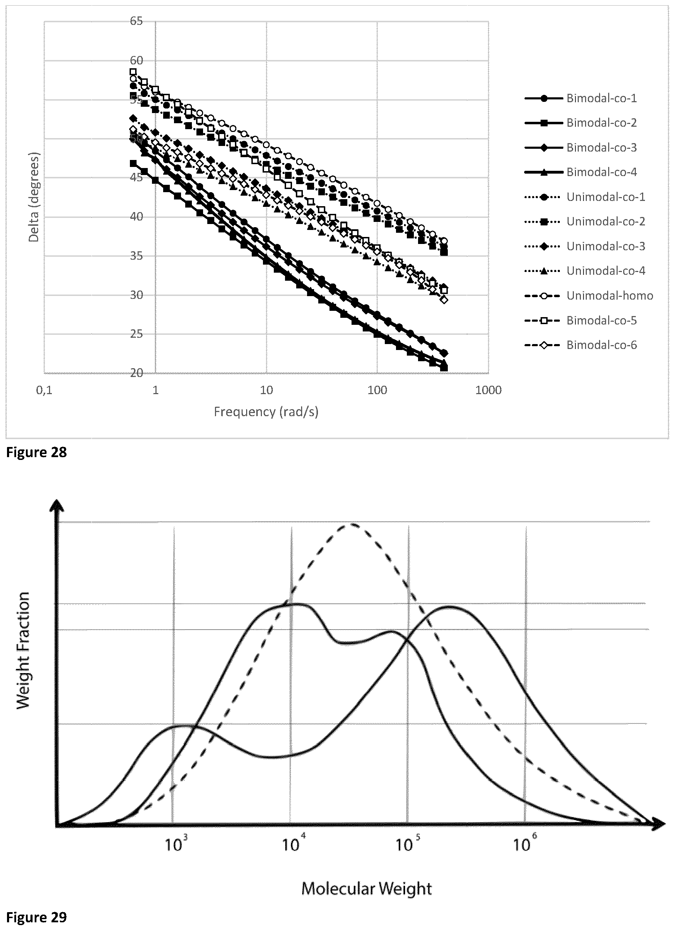

Also, by mixing for example a unimodal molecular weight distribution (Fig. 29) with one of the bimodal molecular weight distributions in Fig. 29, or by mixing the two bimodal molecular weight distributions in Fig. 29, or by mixing all of the molecular weight distributions in Fig. 29 in their melt states during production of carrier media, a carrier media for MBBR having a multimodal molecular weight distribution could be formed. Such a composition may also increase the confidence that the carrier will maintain its integrity through more demanding type of environments. Therefore, a carrier media having a multimodal distribution could be utilized and determined according to ASTM D6474.

Therefore, as described in the present invention a unimodal molecular weight distribution is described as in Fig. 29 having one peak, a bimodal molecular weight distribution is described as in Fig. 29 having two peaks and a multimodal molecular weight distribution is described as having more than two peaks throughout its molecular weight distribution.

In one embodiment, is provided the use of a carrier to carry a biofilm in a moving bed biofilm reactor (MBBR), characterized in that the carrier comprises a carrier material, wherein the carrier material comprises at least one high density polyethylene having a bimodal molecular weight distribution, whereby the carrier material has a multimodal molecular weight distribution.

The carriers of the invention will maintain its integrity through more demanding type of environments, why in one embodiment, the carrier to carry a biofilm in a moving bed biofilm reactor (MBBR) has improved stability against environmental stresses.

In one embodiment, is provided the use of a carrier to carry a biofilm in a moving bed biofilm reactor (MBBR) having improved stability against environmental stresses, characterized in that the carrier comprises a carrier material, wherein the carrier material comprises at least one high density polyethylene having a bimodal molecular weight distribution, whereby the carrier material has a multimodal molecular weight distribution.

The carrier comprising HDPE having a bimodal or multimodal weight distribution has an improved stability against environmental stresses. That means that the carrier will have increased resistance to the long-term damage that was characterized above, that is the brittle rupture due to stress crack formation and propagation that the MBBR carriers suffer during use.

Not only does this extend the carrier lifetime for general MBBR applications, but the increased resistance may facilitate, delay or eliminate refurbishment of older or worn equipment, which has higher wear on the carriers.

It also means that the carrier is more resistant to mixing energies, and may facilitate better use of submersible mixers, or utilization of higher than general mixing energies if necessary.

The improved carrier properties is also an advantage in wastewaters comprising more severe plastic detrimental substances, or in treatment lines for production of drinking water, where production of microplastics coming from the plastic carrier media may be greatly reduced.

Four bimodal HDPE raw materials were utilized for the production of a first carrier media type for MBBR shown in Figure 30. These were compared in terms of lifetime in a first heavy mixing test in a reactor volume containing water and carrier media to carrier materials (Fig. 30) produced from raw materials of unimodal HDPE homopolymer and unimodal HDPE copolymers. Tests were setup to induce elevated shear stress on the carrier media naturally being higher than in a real world situation due to time limitation but still lower than the yield stress of the raw material to avoid plastic deformation and instead induce stress crack formation and brittle fracture.

By mixing for example a HDPE having a unimodal molecular weight distribution with a HDPE having a bimodal molecular weight distribution or for example by mixing two HDPEs with bimodal molecular weight distributions during production of carrier media, a carrier media for MBBR having a multimodal molecular weight distribution could be formed. As according to the present invention a multimodal distribution could be utilized and determined according to ASTM D6474. Utilizing a carrier media for MBBR having a multimodal molecular weight distribution according to above could also be interesting according to the present invention as it may reduce the production price of carrier media without decreasing the stress crack resistance of the carrier media too much. Four batches (three according to the invention and one control) of a second carrier media type for MBBR shown in Fig. 32 comprising a 5 wt.-% bimodal HDPE copolymer and 95 wt.-% unimodal HDPE copolymer, 20 wt.-% bimodal HDPE copolymer and 80 wt.-% unimodal HDPE copolymer, 100% bimodal HDPE copolymer, and 100% unimodal HDPE copolymer (control) were also tested in terms of lifetime in a second heavy mixing test.

### Testing the improved lifetime of modified MBBR plastic carriers versus state of the art MBBR carriers

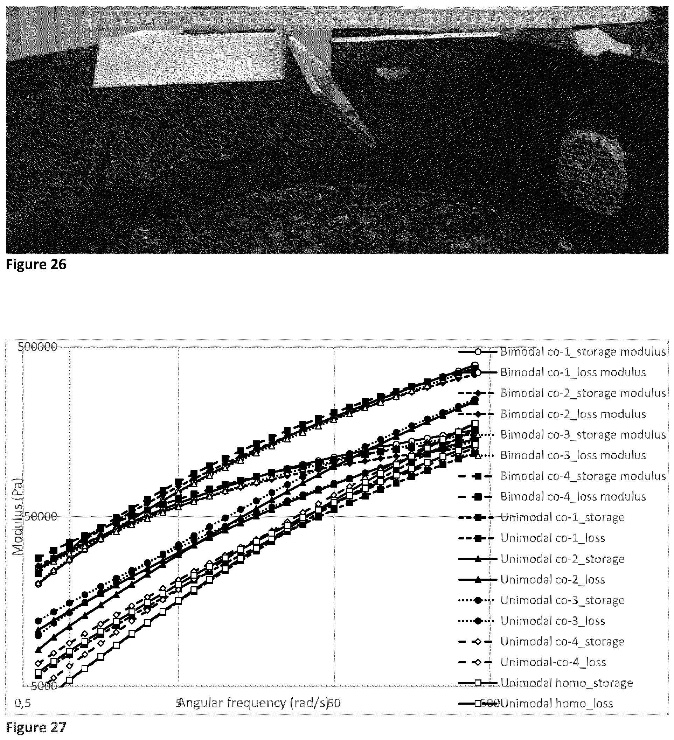

Tests were performed in a cylindrical reactor volume having diameter of 80 cm, length of 150 cm and using a mixer blade according to Fig. 26 with a total blade length of 35 cm. In addition, a board baffle is tightly fastened in the reactor.

The reactor is filled with 340 L of water and 170 L bulk volume of chosen carrier type to get a 50% filling degree by bulk volume of carrier media in relation to water volume. The carriers are first mixed at low speed for 24h to wet the carriers and make them being easily suspended in the water volume upon mixing. The frequency is then elevated having a tip speed of 60 m/s and a power density between 1700-2200 W/m³ where the power density would be 1700 W/m³ if the efficiency is 80%.

This heavy mixing is performed until visible carrier breakage (daily visual inspection of the carriers). The outcome of the tests comparing the time until breakage between a first type of carrier media (as depicted in Fig. 30) comprising different types of bimodal, unimodal HDPE copolymers or homopolymers can be seen in Table 3. Additionally the same heavy mixing tests were performed for a second type of carrier media (as depicted in Fig. 32) comprising blends (mixes) of bimodal and unimodal HDPE copolymers with different amounts of bimodal HDPE incorporated into the blend. The outcomes of these tests can be seen in Table 4.

It was unexpectedly found that even a carrier having as low as 5 wt.% of a first high density polyethylene with a bimodal molecular weight distribution showed a more than 100% prolonged lifetime when compared to a 100% unimodal-copolymer carrier.

As such, the carrier material comprises at least 5 wt. %. such as at least 10, 15, 20, 25, 30, 35, 40 or 45 wt. % of a high density polyethylene with a bimodal molecular weight distribution, whereby the carrier material has a multimodal molecular weight distribution.

The carrier material comprises at least 5 wt. %, such as at least 10, 15, 20, 25, 30, 35, 40 or 45 wt. % of a first high density polyethylene with a bimodal molecular weight distribution, and a second high density polyethylene, the second high density polyethylene having unimodal, bimodal or multimodal molecular weight distribution, *preferably having unimodal* molecular weight distribution, whereby the carrier material has a multimodal molecular weight distribution.

In table 4 is also shown the results for a carrier comprising 20 wt.% of the first high density polyethylene with a bimodal molecular weight distribution. This carrier showed a test lifetime of 28 days compared to the 7 days of the 100% unimodal-copolymer carrier.

In one embodiment, the carrier material comprises at least 50 wt. %, more preferably at least 80 wt. %, most preferably at least 95wt. %, of a high density polyethylene with a bimodal molecular weight distribution.

In one embodiment, the carrier material comprises at least 50 wt. % of a first high density polyethylene with a bimodal molecular weight distribution.

A 100% Bimodal-copolymer showed an astonishing 51 day lifetime compared to the 7 day lifetime for the standard 100% unimodal-copolymer carriers. Such significantly increased resistance to withstand the rough conditions (mimicking those generally employed in different wastewater treatment plants), thereby significantly extends the carrier lifetime.

In one embodiment, the carrier material comprises a first high density polyethylene with a first bimodal molecular weight distribution and a second high density polyethylene with a second bimodal molecular weight distribution.

As such, in one embodiment, the carrier material comprises at least 5 wt.%, such as at least 10, 15, 20, 25, 50, 95 wt.% of a first high density polyethylene with a bimodal molecular weight distribution, and a second high density polyethylene with a bimodal molecular weight distribution.

In one embodiment, the carrier comprises multiple high density polyethylenes with bimodal molecular weight distributions.

In one embodiment, the carrier material comprising high density polyethylene with a bimodal molecular weight distribution, comprises a lower molecular weight fraction (LMW), and a higher molecular weight fraction (HMW), wherein the peak ratio of the LMW fraction to the HMW fraction is between 10:1 to 1:10, preferably between 5:1 to 1:5, more preferably 2.5:1 to 1:2.5, more preferably between 2:1 to 1:2, most preferably between 1.5:1 to 1:1.5.

In one embodiment, the peak ratio is determined according to method ASTM D6474.

Such vastly increased resistance may mean that refurbishment of older or worn equipment of MBBR plants may not be necessary, since the improved properties of the carrier media according to the present invention would be able to handle the rougher conditions for a much extended time compare to state of the art plastic carrier media. This would result in a saving of both resources and cost. Additionally, the improved plastic carriers would increase the scope of possible MBBR applications such as extended utilization of submersible mixers, treatment of wastewater containing substances being detrimental to HDPE properties, utilization of higher mixing energies when necessary, and eliminating and/or minimizing the production of micro-plastics coming from the plastic carriers in the treatment line for production of drinking water.

It is clear from Table 3 that the bimodal-co-1 to 4 carrier media are superior to the carriers comprising bimodal-co-5 to 6 which in turn are superior to the unimodal copolymer carrier media and the unimodal homopolymer carrier media being the one with the shortest lifetime.

It is clear from Table 4 that increasing the amount of bimodal HDPE in the blend with unimodal HDPE significantly increases the lifetime. It is however still of interest to also have MBBR carrier media with lower amounts of bimodal HDPE since it is cheaper to produce meaning lower initial cost for the end-user, however with the price to pay of lower lifetime. The much greater lifetime for carrier media used in Table 4 (Fig. 32) compared to Table 3 (Fig. 30) is because of a different type of lighter carrier media being used in the heavy mixing test in Table 4, thereby generating less shear forces in the reactor and prolonging the lifetime.

Bimodal-1 to Bimodal-6 are various commercially available HDPE co-polymers having a bimodal molecular weight distribution. Unimodal-co-1 to Unimodal-co-4 are various commercially available HDPE co-polymers having a unimodal molecular weight distribution. Unimodal-homo is a commercially available HDPE homopolymer having a unimodal molecular weight distribution.

100% Bimodal-copolymer is a commercially available HDPE co-polymers having a bimodal molecular weight distribution. 100% unimodal-copolymer (control) is a commercially available HDPE co-polymers having a unimodal molecular weight distribution. 5% Bimodal-co-95% unimodal-co and 20% Bimodal-co-80% unimodal-co is a 5:95 wt.% and 20:80 wt.% mix, respectively, of the Bimodal-copolymer and unimodal-copolymer above.

**Table 3: Time until breakage in a first heavy mixing tests of a first MBBR carrier media type comprising HDPE of different molecular structures**

| **Bimodal-1 (hrs)** | **Bimodal-2 (hrs)** | **Bimodal-3 (hrs)** | **Bimodal-4 (hrs)** | **Bimodal-5 (hrs)** | **Bimodal-6 (hrs)** |
|---|---|---|---|---|---|
| (h) | (h) | (h) | (h) | (h) | (h) |
| 459 | 465 | 454 | 462 | 301 | 402 |

| **Unimodal-co-1 (hrs)** | **Unimodal-co-2 (hrs)** | **Unimodal-co-3 (hrs)** | **Unimodal-co-4 (hrs)** | **Unimodal-homo** | |
|---|---|---|---|---|---|
| (h) | (h) | (h) | (h) | (h) | |
| 72 | 105 | 205 | 211 | 27 | |

**Table 4: Time until breakage in a second heavy mixing tests of a second MBBR carrier media type comprising blends of HDPEs of different molecular structures**

| **100% Bimodal-copolymer** | **100% unimodal-copolymer (control)** | **5% Bimodal-co-95% unimodal-co** | **20% Bimodal-co-80% unimodal-co (days)** |
|---|---|---|---|
| | | | |
| 51 days | 7 days | 15 days | 28 days |

### Characterization of the improved MBBR plastic carriers

As was seen in Table 3 some of the bimodal carrier media had longer lifetime in the heavy mixing tests compared to others. Also some unimodal copolymer based carrier media had longer lifetime than other unimodal copolymer based carrier media. It was therefore important to characterize these differences in order to understand the preferred molecular structure of HDPE carrier media according to the present invention. This was done according to the following methods measuring the specific characteristics needed for elongated lifetime as carrier media in different moving bed bioreactor applications.

The storage modulus (G' in Pa) represents the elastic portion of the viscoelastic behavior, which describes the solid-state behavior of the sample. The loss modulus (G" in Pa) characterizes the viscous portion of the viscoelastic behavior, which can be seen as the liquid-state behavior of the sample.

Storage modulus G' represents the stored deformation energy and loss modulus G" characterizes the deformation energy lost (dissipated) through internal friction when flowing. Viscoelastic solids with G' > G" have a higher storage modulus than loss modulus. This is due to links inside the material, for example chemical bonds or physical-chemical interactions. On the other hand, viscoelastic liquids with G" > G' have a higher loss modulus than storage modulus. The reason for this is that, in most of these materials, there are no such strong bonds between the individual molecules.

Therefore, apart from determining the crossover point for characterizing the materials as described in Fig. 6 for further characterization the tangent delta values could be determined as function of the frequency for all materials. The tangent delta values would then compare the materials to see the difference in internal bond strength between them. A higher internal bond strength would be very preferable for MBBR applications.

The tangent delta value describes the ratio of the two portions of the viscoelastic behavior according to below and is calculated by G"/G'. From the tangent delta value, delta value could then be calculated.
1. For ideally elastic behavior δ = 0°. There is no viscous portion. Therefore, G" = 0 and with that tan δ = G" / G' = 0.
2. For ideally viscous behavior δ = 90°. There is no elastic portion. Therefore, G' = 0 and thus the value of tan δ = G"/ G' approaches infinity because of the attempt to divide by zero.

Therefore, the delta value will vary between 0-90°. The lower the delta value the more elastic the behavior of the material is and thereby the stronger the internal chemical bonds or physio-chemical interactions thereby making it more suitable for MBBR applications.

The carrier media tested in the heavy mixing tests were therefore characterized utilizing oscillatory rheology tests to determine the crossover points and delta values for all the materials in Table 3 and 4 according to Method 1 below:

### Method 1:

A TA instruments Discovery HR-2 hybrid rheometer equipped with plate-plate configuration was utilized. 0.4736 g of solid plastic carrier sample was put on the bottom plate with a gap opening between the plates of 25.0 mm in the rheometer before closing the oven chamber and the sample was then allowed to fully melt at 180°C. The chamber was then opened and the molten polymer sample was smeared out and distributed evenly on the bottom plate. The oven was again closed and allowed to reach 180 °C again. The gap between the plates was decreased to 1.0 mm and an oscillatory measurement was carried out according to below.
1. An oscillation time sweep at 180 °C for 600 s at 2.0% strain and a frequency of 10 Hz
2. The oscillation time sweep was directly followed by an oscillation amplitude sweep at 180 °C, a frequency of 1.0 Hz and a logarithmic strain sweep between 0.01 - 100% with 5 points per decade recorded
3. The oscillation amplitude sweep was directly followed by an oscillation frequency sweep 180 °C at a strain of 5.0% and a logarithmic frequency sweep between 0.1-64.0 Hz with 10 points per decade recorded

The crossover point was determined as the intersection of the loss modulus and storage modulus plotted as a function of the angular frequency (from step 3 in method 1).

The oscillatory melt frequency sweep curves data generated after step 3 in Method 1 can be seen in Fig. 27 where the storage and loss modulus was plotted as a function of the angular frequency. The crossover point data were then determined from Fig. 27 to characterize the tested materials and can be seen in Table 5.

Additionally, the delta values were calculated for all materials as function of frequency and the data is shown in in Fig. 28. The range of the delta values for the frequencies tested for each material can also be seen in Table 6.

Density values were measured according to the ASTM 1505 method to make sure that the modified HDPE properties do not dramatically change the density of the carriers which still need to be in the similar range as the state of the art carriers to be possible to utilize in MBBR applications. These results can be seen in Table 7.

The modality of the molecular weight distribution (uni- or bimodal) of the polyethylene was determined according to ASTM D6474 where unimodal and bimodal molecular weight distributions could be defined as the examples according to Fig. 29.

The inclusion of small amounts of co-monomer branching utilizing α-olefins while still maintaining density high enough (>0.94 g/cm³) for utilization in MBBR applications, defines a HDPE copolymer in the present invention while HDPE homopolymers were defined as not containing such co-monomer branching.

In one embodiment, the carrier material has a density of between 0.9-1.1 g/cm³, more preferably a density between 0.92-0.98 g/cm³, even more preferably a density between 0.94-0.96 g/cm³.

The density is determined according to method ASTM D1505.

**Table 5: Values for the crossover points determined by melt rheology frequency sweeps of carrier media (depicted in Fig. 30) comprising either bimodal or unimodal HDPE**

| **COP** | **Bimodal-co-1** | **Bimodal-co-2** | **Bimodal-co-3** | **Bimodal-co-4** | **Bimodal-co-5** | **Bimodal-co-6** |
|---|---|---|---|---|---|---|
| (rad/s) | 2.0 | 0.9 | 1.6 | 1.5 | 12.7 | 4.7 |
| Gx (Pa) | 42700 | 30700 | 37200 | 42000 | 45000 | 25300 |

| **COP** | **Unimodal-co-1** | **Unimodal-co-2** | **Unimodal-co-3** | **Unimodal-co-4** | **Unimodal-homo** | |
|---|---|---|---|---|---|---|
| (rad/s) | 25.0 | 17.3 | 6.4 | 5.9 | 37.4 | |
| G_{X} (Pa) | 40800 | 38200 | 35400 | 27463 | 53349 | |

It is clear from Fig. 27 that MBBR carriers comprising bimodal carrier media (bimodal-co-1 to 4 in Table 4) have angular frequencies at the crossover point that are significantly lower (from about 1 to 1.5) than the MBBR carriers comprising of unimodal HDPE (from about 6 to 40). Additionally, it can be seen in Table 5 that two additional bimodal carrier media (Bimodal-co-5 and 6) can have a higher angular frequencies at the crossover point (from about 5 to 13) but with the consequence of shorter time until breakage in the heavy mixing test as seen in Table 3.

Thus in one embodiment, the carrier material has a crossover point at an angular frequency of <15 rad/s, preferably below 6 rad/s, even more preferably <3 rad/s. The crossover point angular frequency and/or modulus may be determined according to Method 1.

In one embodiment, the carrier material has a crossover point at a modulus between 25000-45000 Pa. The crossover point angular frequency and/or modulus may be determined according to Method 1.

In one embodiment, Method 1 comprises the steps of determining the crossover point angular frequency, modulus and/or delta end value using a TA instruments Discovery HR-2 hybrid rheometer equipped with plate-plate configuration was utilized, wherein 0.4736 g of solid plastic carrier sample was put on the bottom plate with a gap opening between the plates of 25.0 mm in the rheometer before closing the oven chamber and the sample was then allowed to fully melt at 180°C. The chamber was then opened and the molten polymer sample was smeared out and distributed evenly on the bottom plate. The oven was again closed and allowed to reach 180 °C again.The gap between the plates was decreased to 1.0 mm, and an oscillatory measurement was carried out following the steps of: making an oscillation time sweep at 180 °C for 600 s at 2.0% strain and a frequency of 10 Hz, directly followed by an oscillation amplitude sweep at 180 °C, a frequency of 1.0 Hz and a logarithmic strain sweep between 0.01-100% with 5 points per decade recorded, and directly followed by an oscillation frequency sweep 180 °C at a strain of 5.0% and a logarithmic frequency sweep between 0.1-64.0 Hz with 10 points per decade recorded.

At the same time, carrier media unimodal-co 3 and 4 that have had a lower angular frequency at the crossover point compared to the carrier media bimodal-co-5 but still lower lifetime in the heavy mixing test. This shows the importance advantage of the bimodality with co-monomer incorporated in the higher molecular weight part. It is also seen in Table 5 that carrier media unimodal-co-1 and 2 have significantly higher angular frequencies at the crossover point (lower average molecular weight) compared to carrier media unimodal-co-3 and 4 and therefore shorter lifetime (lower molecular weight) in the heavy mixing test as seen in Table 3. Finally, the unimodal homopolymer with showed the by far highest angular frequency at the crossover point and no co-monomer included had the shortest lifetime in the heavy mixing test (Table 3).

This clearly demonstrated the importance of bimodality versus unimodality and also the importance advantage of a higher molecular weight both for different bimodal carrier media but also for unimodal carrier media. Bimodality with inclusion of copolymer in the high molecular weight part only has the highest effect on higher stress cracking resistance followed by a higher angular frequency at the crossover point (higher average molecular weight). The higher stress crack resistance gives the carrier media a longer process lifetime or the ability to withstand harsher conditions for a significantly longer time in MBBR applications.

In one embodiment, the carrier material comprises high density polyethylene with a bimodal molecular weight distribution, comprising a lower molecular weight fraction (LMW), and a higher molecular weight fraction (HMW), wherein the LMW is a homopolymer or a copolymer and the HMW is a homopolymer or copolymer; preferably the LMW being a homopolymer and the HMW being a copolymer.

Furthermore, crossover points of the carrier media (depicted in Fig. 32) tested in the heavy mixing tests in Table 4 comprising blends of unimodal and different amounts of bimodal HDPE were determined. The values of the crossover points of these blends are shown in Table 6.

**Table 6: Values for the crossover points determined by melt rheology frequency sweeps of carrier media (depicted in Fig. 32) comprising 100% bimodal HDP, 100% unimodal HDPE copolymer, a blend of 5% bimodal-95% unimodal HDPE or a blend of 20% bimodal-80% unimodal HDPE**

| **COP** | **100% Bimodal-copolymer** | **100% unimodal-copolymer (ctrl)** | **5% Bimodal-co-95% unimodal-co** | **20% Bimodal-co-80% unimodal-co** |
|---|---|---|---|---|
| (rad/s) | 1.5 | 17.3 | 9.8 | 4,7 |
| G_{X} (Pa) | 42000 | 38200 | 39500 | 31000 |

It is clear from Table 6 that the more bimodal HDPE the carrier media comprises the lower the angular frequency at the crossover point indicating higher average molecular weight of the blend when increasing the bimodal amount in the carrier media. This fits well with the increase in time until breakage for these carrier media as shown in Table 4. Thereby utilizing a 100% pure bimodal carrier media is preferable in terms of performance, however the carrier media can be improved also with smaller inclusions of bimodal HDPE. Since bimodal HDPE generally is more expensive to produce than unimodal HDPE utilizing a smaller amount than 100% pure bimodal HDPE by utilizing a blend of bimodal and unimodal HDPE or even blending different bimodal HDPE material could be interesting for certain applications.

In one embodiment, the carrier material has a delta end value <35 degrees more preferably <25 degrees, more preferably <20 degrees, even more preferably <15 degrees. The delta end value may be determined according to Method 1.

It is clear from the data in Fig. 28 and Table 7 that MBBR carriers comprising the bimodal-co-1 to 4 carrier media have significantly lower delta values throughout the frequency range compared to the carriers comprising bimodal-co-5 and 6 carrier media which had similar delta values to the unimodal-co-3 and 4 carrier media, especially in the higher frequency range when higher shear is induced on the sample. The unimodal-co-1 and 2 carrier media had higher delta values throughout the frequency range compared to the above mentioned and the unimodal-homo carrier media had the lowest delta values. This indicated that the carriers comprising the bimodal-co-1 to 4 carrier media with the lowest angular frequencies at the crossover point were more elastic in the melt at higher frequencies compared to the bimodal-co-5 and 6 and the unimodal samples. Bimodal-co-5 carrier media although having a higher angular frequency (lower average molecular weight) at the crossover point compared to the unimodal-co-3 and 4 samples had similar delta values in the high frequency range again showing that bimodality with co-monomer incorporated in the high molecular weight part can offset a lower average molecular weight compared to unimodal copolymers. A lower delta value at higher frequencies meant the internal links inside the material, for example chemical bonds or physical-chemical interactions were stronger. This also underlined the significant improvement in properties of the bimodal carrier media compared to the unimodal carrier media and also the further improvement if increasing the molecular weight of bimodal carrier media thereby significantly improving its lifetime in MBBR processes.

**Table 7: Delta start and end values of carrier media (as depicted according to Fig. 30) comprising different bimodal or unimodal HDPE determined by melt rheology frequency sweeps**

| **Delta value** | **Bimodal-1** | **Bimodal-2** | **Bimodal-3** | **Bimodal-4** | **Bimodal-5** | **Bimodal-6** |
|---|---|---|---|---|---|---|
| δ start (°) | 50.9 | 46.8 | 50.0 | 51.0 | 58.5 | 51.2 |
| δ end (°) | 22.6 | 20.6 | 22.5 | 21.3 | 30.6 | 29.4 |

| **Delta value** | **Unimodal-co-1** | **Unimodal-co-2** | **Unimodal-co-3** | **Unimodal-co-4** | **Unimodal-homo** | |
|---|---|---|---|---|---|---|
| δ start (°) | 56.8 | 55.5 | 52.6 | 52.0 | 57.7 | |
| δ end (°) | 36.2 | 35.4 | 31.0 | 31.5 | 36.9 | |

As seen in Table 8 the delta end value is lowered in the bimodal-unimodal blends with increased bimodal content indicating stronger internal linkages in the blends compared to the pure unimodal carrier media.

**Table 8: Delta start and end values of carrier media (as depicted according to Fig. 32) comprising bimodal, unimodal or different blends of bimodal and unimodal HDPE determined by melt rheology frequency sweeps.**

| **Delta value** | **Bimodal-100%** | **Unimodal-100%** | **Bimodal 5%-Unimodal 95%** | **Bimodal 20%-unimodal 80%** |
|---|---|---|---|---|
| δ start (°) | 51.0 | 55.5 | 55.5 | 52.5 |
| δ end (°) | 21.3 | 35.4 | 33.4 | 31.1 |

As according to Table 9 most of the carriers comprising bimodal HDPE have slightly lower density than the carriers comprising unimodal HDPE, however still well within range for being suitable for MBBR applications.

**Table 9: Density of the different HDPE carrier materials determined by ASTM 1505**

| **Density** | **Bimodal-1** | **Bimodal-2** | **Bimodal-3** | **Bimodal-4** | **Bimodal-5** | **Bimodal-6** |
|---|---|---|---|---|---|---|
| (g/cm³) | 0.949 | 0.956 | 0.949 | 0.949 | 0.946 | 0.959 |

| **Density** | **Unimodal-co-1** | **Unimodal-co-2** | **Unimodal-co-3** | **Unimodal-co-4** | **Unimodal-homo** | |
|---|---|---|---|---|---|---|
| (g/cm³) | 0.959 | 0.951 | 0.955 | 0.955 | 0.964 | |

As according to Fig. 29 the molecular weight distribution of different HDPEs can be either unimodal or bimodal with the unimodal molecular weight distribution showing one peak throughout the distribution while the bimodal distribution shows two peaks throughout the molecular weight distribution. As according to the present invention a bimodal distribution is generally utilized and determined according to ASTM D6474.

### Use of plastic carrier media with improved stability against mechanical stress in different MBBR applications

The carrier media for MBBR with improved stability against environmental stresses will be of importance to significantly increase the lifetime of the plastic carrier media in the general MBBR applications, improving end-user value in terms of cost efficiency and treatment performance but in addition also giving other environmental benefits such eliminating micro-plastic pollution from worn carrier media over time and decreasing CO₂ footprint for production of carrier media thanks to longer lifetime.

The invention is the use of a carrier in a moving bed biofilm reactor (MBBR) process.

MBBR processes are well known in the art and are described in several publications, such as H. Odegaard ET AL (1994). In short, MBBR is a method for water purification in which wastewater is fed into a reactor containing carriers having a biofilm which promotes a desired conversion of impurities. Carriers are traditionally made of extruded or injection molded plastics and usually share properties such as having a larger surface than smooth elements of the same dimension (often having a growth surface up to 100 times larger than a corresponding smooth object) and having a density in the range 0.90 to 1.20, normally 0.92 till 0.98, particularly 0.94 to 0.96 kg/dm³. Lately, MBBR carriers have moved towards being bigger in size and having more complicated substructures in order to increase its specific protected surface area, often having a protected surface area of 500 m²/m³ or larger, to improve process performance and to be able to lower needed reactor tank volume.

MBBR carriers with biofilm are kept suspended in the water in a reactor for aerobic, anoxic or anaerobic water purification. Such reactors typically comprises inlet and outlet tubes and sieve strainers retaining the carrier in the reactor and optionally mixing means, and containing a large number of carriers having biofilm formation on its protected surface. The carriers will thus repeatedly collide with each other, repeatedly colliding with reactor walls and with other equipment inside the reactor upon being suspended in the reactor volume by utilization of air and/or mechanical mixers, causing significant shear stresses to continuously act on the carriers.

In the invention, MBBR carrier were found to be particularly sensitive to stress damage (as shown in figures 8 to 22) and it was found that MBBR carriers having a carrier material with a bimodal or a multimodal molecular weight distribution gains improved stability against environmental stresses in specific MBBR applications. This becomes especially apparent when using larger carriers and processes with lower reactor tank volumes, since larger sized carriers and lower tank volumes will increase the shear forces acting on each particular carrier. Similarly, carriers with complicated substructures are likely to make the carrier weaker in terms of handling these higher shear forces without breaking.

In one embodiment, the carrier has a protected surface area of at least 200 m²/m³, preferably at least 300 m²/m³, more preferably at least 400 m²/m³, such as at least 500 m²/m³, such as at least 600 m²/m³, such as at least 800 m²/m³, or a protected surface area of 200 to 1200 m²/m³, such as 300 to 1100 m²/m³, such as 300 to 1000 m²/m³.

In one embodiment, the use of a carrier is in a MBBR process to purify a liquid from contaminants, the MBBR process utilizing a MBBR system comprising at least one bioreactor, the bioreactor being continuously or intermittently aerated and/or mixed, wherein the carrier is retained within the at least one bioreactor, the carrier being kept in suspension and in movement, either continuously or intermittently, in the liquid to be purified by aeration and/or mixing of the liquid to be purified, and wherein the carrier provides a protected surface for biofilm growth.

Further disclosed herein is a method to purify a liquid from contaminants utilizing a MBBR system, comprising the steps of:
continuously or intermittently adding the liquid to be purified to a bioreactor comprising and retaining MBBR carriers with improved resistance,
continuously or intermittently aerating and/or mixing the liquid, thereby keeping the carriers in suspension and in movement,
continuously or intermittently removing purified liquid from the at least one bioreactor,
wherein the carrier provides a protected surface for biofilm growth of microorganisms which feed on the contaminants in the liquid to be purified.

In one embodiment, the carrier having a structure that allows for protected surfaces for biofilm growth, wherein the protected surfaces are facilitated by the presence of holes, wells, protrusions, honeycomb structure or raster structures, [made from thin material] making the carrier more prone to stress damage.

However, some specific MBBR applications would benefit even more than the general MBBR application from utilizing the carrier media according to the present invention. These specific applications are listed below.

### - High energy MBBR applications

For MBBR applications where high mixing or aeration energy is needed the shear forces in the bio-reactor, and therefore the shear forces applied to the carrier media, are significantly higher than for MBBR applications where lower mixing or aeration energy is utilized. These higher shear forces acting on the media shortens its lifetime until breakage, forcing the operator of the plant to replace the carrier media in a significantly shorter time than normal at significant cost. Additionally, not replacing the carrier media in time can cause broken plastics pieces to pass through sieves, degrade biological treatment performance and release plastic into nature in terms of micro- and macro-plastic pollution. By utilizing carrier media according to the present invention, operators of wastewater treatment plants utilizing MBBR applications with high mixing and/or aeration energy can avoid unnecessary cost, avoid potential release of micro- and macro-plastics and decrease its CO₂ footprint due to the greatly improved properties and lifetime of the carrier media.

While shear forces will depend on several factors such as the reactor layout, it is intrinsically linked to the reactor size and the kinetic energy of the carriers. For a mechanically mixed reactor, a supplied stirring energy of at least 15 W/m³ reactor volume is generally considered a high energy MBBR application. Similarly, an aerated reactor having a supplied effect of aeration energy of at least 40 W/m³ reactor volume is generally considered a high energy application.

In one embodiment, the moving bed biofilm reactor (MBBR) process is a High energy MBBR process. In one further embodiment, the high energy process has a stirring energy of at least 15 W/m³ reactor volume or an aeration energy of at least 40 W/m³ reactor volume.

### Submersible mixer applications

MBBR plants utilizing submersed mixers generally assert higher shear stress to MBBR carriers when in motion compared to top-mounted mixers. Therefore utilization of such mixers, which in some wastewater treatment plants are necessary due to design and site limitations, may affect the carrier media lifetime negatively. Therefore utilizing the carrier media according to the present invention in such plants would greatly enhance carrier media lifetime despite the use of submersible mixers.

In one embodiment, the MBBR process includes mixing the carriers in the bioreactor utilizing submerged mechanical mixers.

### MBBR retrofits of existing MBBR plants with poor reactor wall conditions

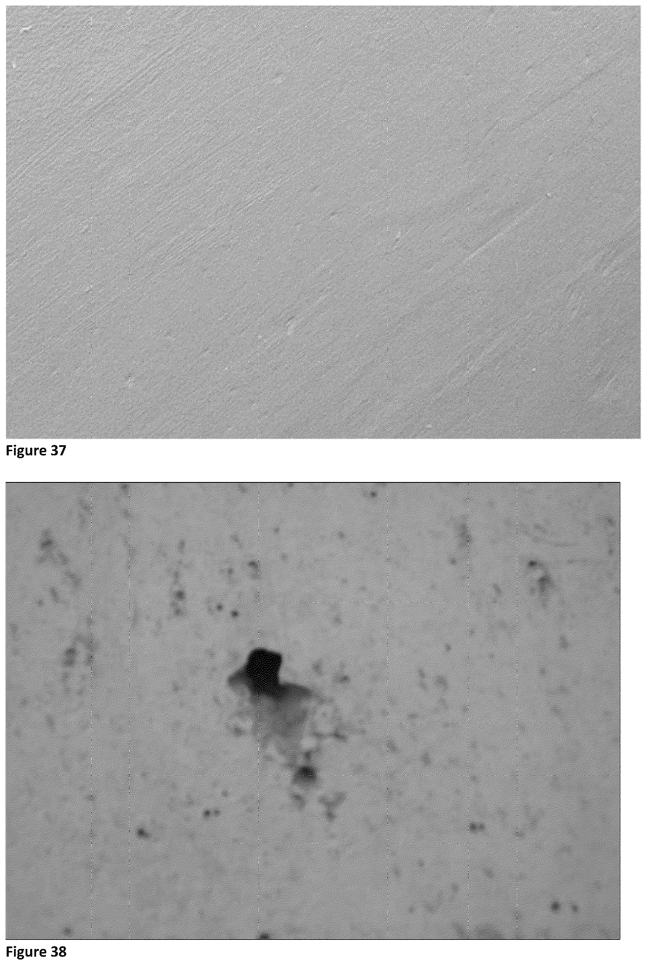

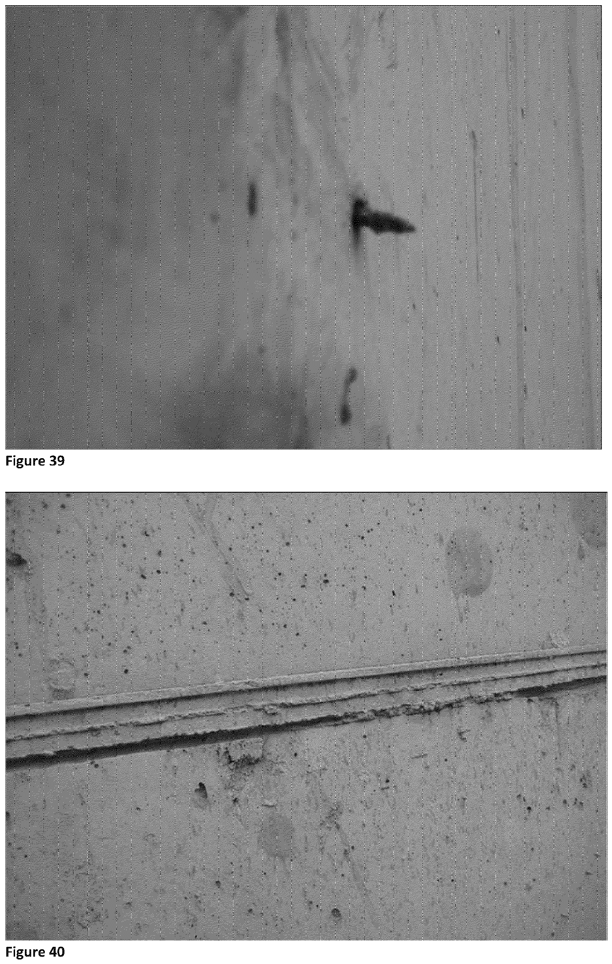

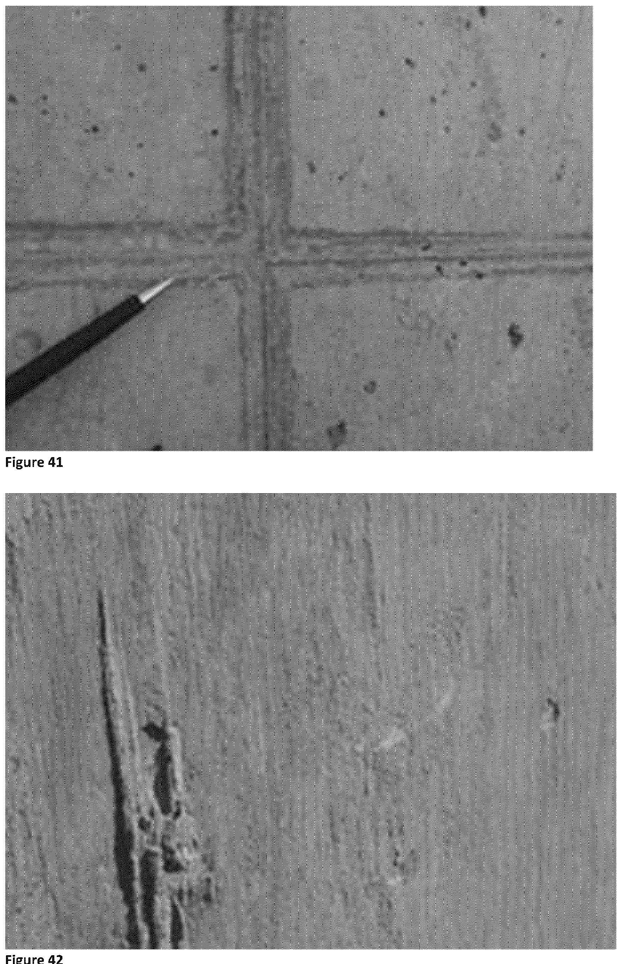

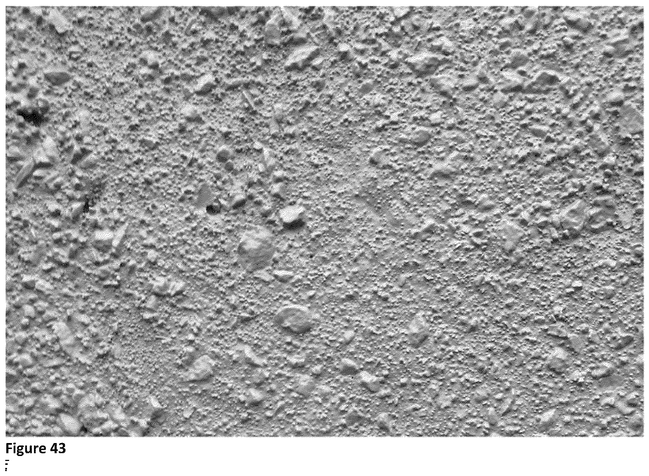

Retrofitting existing wastewater treatment plants and its infrastructure with average concentrations of municipal sewage is generally necessary when organic loading rates to the plant increase. To still be able to meet the effluent limits the plant needs to be up-graded or retrofitted. Retrofitting may involve adding MBBR carrier media to existing reactor volumes to increase treatment capacity. Generally, a newly built and non-utilized MBBR bioreactors would have smooth inner walls as depicted in Fig. 38. Existing already utilized volumes may have poor inner reactor walls that have deteriorated with time due to wear causing as for example, but not limited to, wall cavities (Fig. 39), sharp objects sticking out (Fig. 40), rough joints (Fig. 41 and 42), imperfect grades (Fig. 43) or more severe wall degradation (Fig. 44). All these types of defects or deteriorations cause increased shear stresses to carrier media being kept in aeration when they hit the reactor walls thereby reducing their lifetime.

Generally, the reactor walls must be smooth not to cause enhanced wear of the carrier media. The inside walls of the concrete tanks should have the smoothness that is normally achieved using steel forms and joint ridges should be grinded smooth. Restauration of worn rough walls to a smooth inner wall structure is expensive and would also mean that the reactor would have to be taken out of service during the restauration. Instead, utilizing the carrier media of the present invention having significantly improved stability against environmental stresses, wall restauration may be avoided with the carrier media still able to withstand the rough wall structures during operation.

In one embodiment, the MBBR process utilizes a bioreactor having non-smooth inner walls with defects or high roughness, such as in MBBR retrofits or existing MBBR plants with poor reactor wall conditions.

### MBBR applications where the wastewater contains hazardous substances being detrimental on the carrier media

The stress cracking behavior and time to brittle failure of the carrier media under stress can be further shortened if exposed to stress cracking agents. Stress cracking agents, such as for example solvents or detergents, act to lower the cohesive forces which maintain the tie molecules in the crystallites, thus facilitating faster disentanglement from the crystalline lamellae region. As a result, stress cracking is initiated at even lower stress values. Since wastewater and especially more concentrated industrial wastewater streams may contain a number of such stress cracking agents, either in lower or higher concentrations, utilizing the carrier media according to the present invention will significantly increase the resistance to such stress cracking agents thereby significantly enhancing the lifetime of the carrier media. In some industrial applications where the wastewater is highly contaminated with substances having detrimental effects on the molecular and/or physical stability of state of the art plastic MBBR carrier media, the improved stability of the plastic MBBR carrier media according to the present invention will mitigate that detrimental effect.

In one embodiment, wastewater purified in the MBBR process contains hazardous substances being detrimental on the carrier.

### MBBR applications where external chemical substance additions are made

In many biological wastewater treatment plants external carbon sources such as methanol or ethanol are added to certain biological reactors in order to facilitate denitrification in the wastewater. Such carbon sources may act as stress cracking agents as described above shortening the time until stress crack formation in the carrier media and thereby its lifetime. For such applications utilizing the carrier media according to the present invention would be able to mitigate such detrimental effect and significantly increase the carrier media lifetime in environments and applications where such external carbon source additions are made.

In one embodiment, the MBBR process function is dependent on addition of external chemical substances such as for example, but not limited to, external carbon sources.

### MBBR as part of the treatment line for production of drinking water applications

Utilizing MBBR as part of the treatment line for production of drinking water is an emerging technology. Utilizing MBBR treatment for production of drinking water does not produce any significant amount of surplus suspended sludge in the reactor since almost all of the biomass is formed as a biofilm on the carrier media since the water to be treated in order to produce drinking water is very dilute and does not contain as much pollutants as wastewater. Any possible deterioration of carrier media due to shear stresses in the MBBR reactor generating microplastics may therefore be transferred into the effluent from the treatment process and go further into the treatment line and the produced drinking water. For general MBBR applications for wastewater treatment this possible microplastics contamination gets stuck in the suspended sludge which is then separated from the treated effluent thereby not allowing possible microplastic contamination in the effluent. In the treatment line for production of microplastics this is however not necessarily the case since very little suspended solids are produced due to the diluted water and any possible microplastic poluution coming from the carrier media may proceed further down in the treatment line. Utilizing the carrier media of the present invention microplastics formation will not take place due to the significantly enhanced properties of the carrier media thereby making the carrier media of the present invention a good fit for use in MBBR applications as part of the treatment line in the production of drinking water.

In one embodiment, the MBBR process is utilized as a treatment for the production of drinking water.

Although the present invention has been described above with reference to (a) specific embodiment(s), it is not intended to be limited to the specific form set forth herein. Rather, the invention is limited only by the accompanying claims and, other embodiments than the specific above are equally possible within the scope of these appended claims, e.g. different than those described above.

In the claims, the term "comprises/comprising" does not exclude the presence of other elements or steps. Furthermore, although individually listed, a plurality of means, elements or method steps may be implemented by e.g. a single unit or processor. addition, singular references do not exclude a plurality. The terms "a", "an", "first", "second" etc do not preclude a plurality. Reference signs in the claims are provided merely as a clarifying example and shall not be construed as limiting the scope of the claims in any way.

### References

Brown, N. & Ward, I. M. (1983), "The influence of morphology and molecular weight on ductile-brittle transitions in linear polyethylene", Journal of Materials Science, vol. 18, pp. 1405-1420.
Huang, Y. & Brown, N. (1988), "The effect of molecular weight on slow crack growth in linear polyethylene homopolymers", Journal of Materials Science, vol. 23, pp. 3648-3655.
Odegaard H. ET AL: "A new moving bed biofilm reactor - applications and results", Water Science and Technology, 1 October 1994 (1994-10-01).
Janimak, J. J. & Stevens, G. C. (2001), "Inter-relationships between tie-molecule concentration, molecular characteristics and mechanical properties in metallocene catalysed medium density polyethylenes", Journal of Materials Science, vol. 36, no. 8, pp. 1879-1884.
Yeh, J. T. & Runt, J. (1991), "Fatigue crack propagation in high-density polyethylene", Journal of Polymer Science, Part B: Polymer Physics, vol. 29, pp. 371-388.

## Claims

1. Use of a carrier to carry a biofilm in a moving bed biofilm reactor (MBBR) in a moving bed biofilm reactor (MBBR) process, **characterized in that** the carrier comprises a carrier material,
wherein
the carrier material comprises at least 5 wt. %, such as at least 10, 15, 20, 25, 30, 35, 40 or 45 wt. % of a first high density polyethylene with a bimodal molecular weight distribution, and a second high density polyethylene, the second high density polyethylene having unimodal, bimodal or multimodal molecular weight distribution, *preferably having unimodal* molecular weight distribution, whereby the carrier material has multimodal molecular weight distribution.

2. Use according to claim 1, wherein the carrier material has a density between 0.9 and 1.1 g/cm³, such as a density between 0.92 and 0.98 g/cm³, such as a density between 0.94 and 0.96 g/cm³.

3. Use according to any one of claims 1 to 2, wherein the carrier material comprises
at least 50 wt. %, more preferably at least 80 wt. %, most preferably at least 95wt. %, of a high density polyethylene with a bimodal molecular weight distribution.

4. Use according to any one of one claims 1 to 3, wherein the carrier material comprises
at least 50 wt. % of a first high density polyethylene with a bimodal molecular weight distribution.

5. Use according to any one of claims 1 to 4, wherein the carrier material comprises
a first high density polyethylene with a first bimodal molecular weight distribution and a second high density polyethylene with a second bimodal molecular weight distribution.

6. Use according to claim 5, wherein the carrier material comprises
at least 5 wt.%, such as at least 10, 15, 20, 25, 50, 95 wt.% of a first high density polyethylene with a bimodal molecular weight distribution, and
a second high density polyethylene with a bimodal molecular weight distribution.

7. Use according to any one of claim 1 to 6, the carrier material comprising high density polyethylene with a bimodal molecular weight distribution, comprising
a lower molecular weight fraction (LMW), and
a higher molecular weight fraction (HMW),
wherein the peak ratio of the LMW fraction to the HMW fraction is between 10:1 to 1:10, preferably between 5:1 to 1:5, more preferably 2.5:1 to 1:2.5, more preferably between 2:1 to 1:2, most preferably between 1.5:1 to 1:1.5.

8. Use according to any one of claims 1 to 7, the carrier material comprising high density polyethylene with a bimodal molecular weight distribution, comprising
a lower molecular weight fraction (LMW), and
a higher molecular weight fraction (HMW),
wherein the LMW is a homopolymer or a copolymer and the HMW is a homopolymer or copolymer; preferably the LMW being a homopolymer and the HMW being a copolymer.

9. Use according to any one of claims 1 to 8, wherein the carrier has a structure that allows for protected surfaces for biofilm growth.

10. Use according to claim 9, wherein the carrier is disc-shaped or saddle shaped.

11. Use according to claim 9 or 10, wherein the protected surfaces are facilitated by the presence of holes, wells, protrusions, honeycomb structure or raster structures, making the carrier more prone to stress damage.

12. Use according to any one of claims 1 to 11, wherein the MBBR process is a High energy MBBR process having a stirring energy of at least 15 W/m³ reactor volume or an aeration energy of at least 40 W/m³ reactor volume.

13. Use according to any one of claims 1 to 12, wherein the MBBR process includes mixing the carriers in the bioreactor utilizing submerged mechanical mixers.

14. Use according to any one of claims 1 to 13, wherein wastewater purified in the MBBR process contains hazardous substances being detrimental to the carrier.

15. Use according to any one of claims 1 to 14, wherein the MBBR process function is dependent on addition of external chemical substances, such as external carbon sources.

16. Use according to any one of claims 1 to 15, wherein the MBBR process is a treatment for the production of drinking water.

## Patentansprüche

1. Verwendung eines Trägers zum Tragen eines Biofilms in einem Bewegtbett-Biofilmreaktor (MBBR; engl.: moving bed biofilm reactor) in einem Bewegtbett-Biofilmreaktor (MBBR)-Verfahren, **dadurch gekennzeichnet, dass** der Träger ein Trägermaterial umfasst,
wobei das Trägermaterial mindestens 5 Gew.-%, wie z.B. mindestens 10, 15, 20, 25, 30, 35, 40 oder 45 Gew.-% eines ersten High-Density-Polyethylens mit einer bimodalen Molekulargewichtsverteilung und ein zweites High-Density-Polyethylen umfasst, wobei das zweite High-Density-Polyethylen eine unimodale, bimodale oder multimodale Molekulargewichtsverteilung, *bevorzugt eine unimodale* Molekulargewichtsverteilung, aufweist, wodurch das Trägermaterial eine multimodale Molekulargewichtsverteilung aufweist.

2. Verwendung gemäß Anspruch 1, wobei das Trägermaterial eine Dichte zwischen 0,9 und 1,1 g/cm³, wie z.B. eine Dichte zwischen 0,92 und 0,98 g/cm³, wie z.B. eine Dichte zwischen 0,94 und 0,96 g/cm³, aufweist.

3. Verwendung gemäß einem der Ansprüche 1 bis 2, wobei das Trägermaterial
mindestens 50 Gew.-%, besonders bevorzugt mindestens 80 Gew.-%, am meisten bevorzugt mindestens 95 Gew.-%, eines High-Density-Polyethylens mit einer bimodaler Molekulargewichtsverteilung umfasst.

4. Verwendung gemäß einem der Ansprüche 1 bis 3, wobei das Trägermaterial
mindestens 50 Gew.-% eines ersten High-Density-Polyethylens mit einer bimodalen Molekulargewichtsverteilung umfasst.

5. Verwendung gemäß einem der Ansprüche 1 bis 4, wobei das Trägermaterial
ein erstes High-Density-Polyethylen mit einer ersten bimodalen Molekulargewichtsverteilung und ein zweites High-Density-Polyethylen mit einer zweiten bimodalen Molekulargewichtsverteilung umfasst.

6. Verwendung gemäß Anspruch 5, wobei das Trägermaterial
mindestens 5 Gew.-%, wie z.B. mindestens 10, 15, 20, 25, 50, 95 Gew.-%, eines ersten High-Density-Polyethylens mit einer bimodalen Molekulargewichtsverteilung, und
ein zweites High-Density-Polyethylen mit einer bimodalen Molekulargewichtsverteilung umfasst.

7. Verwendung gemäß einem der Ansprüche 1 bis 6, wobei das Trägermaterial High-Density-Polyethylen mit einer bimodalen Molekulargewichtsverteilung, umfassend
eine Fraktion mit geringerem Molekulargewicht (LMW; engl.: lower molecular weight fraction), und
eine Fraktion mit höherem Molekulargewicht (HMW; engl.: higher molecular weight fraction),
umfasst,
wobei das Peak-Verhältnis der LMW-Fraktion zu der HMW-Fraktion zwischen 10:1 bis 1:10 liegt, bevorzugt zwischen 5:1 bis 1:5, besonders bevorzugt 2,5:1 bis 1:2,5, besonders bevorzugt zwischen 2:1 bis 1:2, am meisten bevorzugt zwischen 1,5:1 bis 1:1,5.

8. Verwendung nach einem der Ansprüche 1 bis 7, wobei das Trägermaterial High-Density-Polyethylen mit einer bimodalen Molekulargewichtsverteilung, umfassend
eine Fraktion mit geringerem Molekulargewicht (LMW) und
eine Fraktion mit höherem Molekulargewicht (HMW),
umfasst,
wobei die LMW ein Homopolymer oder ein Copolymer ist und die HMW ein Homopolymer oder Copolymer ist; bevorzugt die LMW ein Homopolymer ist und die HMW ein Copolymer ist.

9. Verwendung gemäß einem der Ansprüche 1 bis 8, wobei der Träger eine Struktur, die geschützte Oberflächen für das Biofilmwachstum ermöglicht, aufweist.

10. Verwendung gemäß Anspruch 9, wobei der Träger scheibenförmig oder sattelförmig ist.

11. Verwendung gemäß Anspruch 9 oder 10, wobei die geschützten Oberflächen durch das Vorhandensein von Löchern, Vertiefungen, Vorwölbungen, Wabenstrukturen oder Rasterstrukturen begünstigt werden, wodurch der Träger anfälliger für Spannungsschäden wird.

12. Verwendung gemäß einem der Ansprüche 1 bis 11, wobei das MBBR-Verfahren ein Hochenergie-MBBR-Verfahren, das eine Rührenergie von mindestens 15 W/m³ Reaktorvolumen oder eine Belüftungsenergie von mindestens 40 W/m³ Reaktorvolumen aufweist, ist.

13. Verwendung gemäß einem der Ansprüche 1 bis 12, wobei das MBBR-Verfahren das Mischen der Trägerstoffe in den Bioreaktor unter Verwendung von mechanischen Tauchmixern einschließt.

14. Verwendung gemäß einem der Ansprüche 1 bis 13, wobei das im MBBR-Verfahren gereinigte Abwasser gefährliche Substanzen enthält, die für den Träger schädlich sind.

15. Verwendung gemäß einem der Ansprüche 1 bis 14, wobei die Funktion des MBBR-Prozesses von der Zugabe externer chemischer Substanzen, wie z. B. externer Kohlenstoffquellen, abhängig ist.

16. Verwendung gemäß einem der Ansprüche 1 bis 15, wobei das MBBR-Verfahren eine Aufbereitung zur Erzeugung von Trinkwasser ist.

## Revendications

1. Utilisation d'un support pour transporter un film biologique dans un réacteur à film biologique à lit mobile (MBBR) dans un processus par réacteur à film biologique à lit mobile (MBBR), **caractérisée en ce que** le support comprend un matériau de support,
dans laquelle
le matériau de support comprend au moins 5 % en poids, tel qu'au moins 10, 15, 20, 25, 30, 35, 40 ou 45 % en poids, d'un premier polyéthylène haute densité ayant une distribution de poids moléculaire bimodale, et d'un second polyéthylène haute densité, le second polyéthylène haute densité ayant une distribution de poids moléculaire unimodale, bimodale ou multimodale, de préférence ayant une distribution de poids moléculaire unimodale, ce qui permet au matériau de support d'avoir une distribution de poids moléculaire multimodale.

2. Utilisation selon la revendication 1, dans laquelle le matériau de support a une densité comprise entre 0,9 et 1,1 g/cm3, telle qu'une densité comprise entre 0,92 et 0,98 g/cm³, telle qu'une densité comprise entre 0,94 et 0,96 g/cm³.

3. Utilisation selon l'une quelconque des revendications 1 et 2, dans laquelle le matériau de support comprend
au moins 50 % en poids, de manière plus préférée au moins 80 % en poids, de manière la plus préférée au moins 95 % en poids, d'un polyéthylène haute densité ayant une distribution de poids moléculaire bimodale.

4. Utilisation selon l'une quelconque des revendications 1 à 3, dans laquelle le matériau de support comprend
au moins 50 % en poids d'un premier polyéthylène haute densité ayant une distribution de poids moléculaire bimodale.

5. Utilisation selon l'une quelconque des revendications 1 à 4, dans laquelle le matériau de support comprend
un premier polyéthylène haute densité ayant une première distribution de poids moléculaire bimodale et un second polyéthylène haute densité avec une seconde distribution de poids moléculaire bimodale.

6. Utilisation selon la revendication 5, dans laquelle le matériau de support comprend
au moins 5 % en poids, tel qu'au moins 10, 15, 20, 25, 50, 95 % en poids, d'un premier polyéthylène haute densité ayant une distribution de poids moléculaire bimodale, et
un second polyéthylène haute densité ayant une distribution de poids moléculaire bimodale.

7. Utilisation selon l'une quelconque des revendications 1 à 6, le matériau de support comprenant un polyéthylène haute densité ayant une distribution de poids moléculaire bimodale, comprenant
une fraction de poids moléculaire inférieur (LMW), et
une fraction de poids moléculaire supérieur (HMW),
dans laquelle le rapport maximal entre la fraction LMW et la fraction HMW est compris entre 10:1 et 1:10, de préférence entre 5:1 et 1:5, de manière plus préférée entre 2,5:1 et 1:2,5, de manière plus préférée entre 2:1 et 1:2, de manière la plus préférée entre 1,5:1 et 1:1,5.

8. Utilisation selon l'une quelconque des revendications 1 à 7, le matériau de support comprenant un polyéthylène haute densité ayant une distribution de poids moléculaire bimodale, comprenant
une fraction de poids moléculaire inférieur (LMW), et
une fraction de poids moléculaire supérieur (HMW),
dans laquelle la fraction LMW est un homopolymère ou un copolymère et la fraction HMW est un homopolymère ou un copolymère ; de préférence la fraction LMW étant un homopolymère et la fraction HMW étant un copolymère.

9. Utilisation selon l'une quelconque des revendications 1 à 8, dans laquelle le support présente une structure qui permet des surfaces protégées pour une croissance de film biologique.

10. Utilisation selon la revendication 9, dans laquelle le support est en forme de disque ou en forme de selle.

11. Utilisation selon la revendication 9 ou 10, dans laquelle les surfaces protégées sont facilitées par la présence de trous, de puits, de saillies, d'une structure en nid d'abeilles ou de structures de quadrillage, rendant le support plus propice à un dommage par contraintes.

12. Utilisation selon l'une quelconque des revendications 1 à 11, dans laquelle le processus MBBR est un processus MBBR à haute énergie ayant une énergie d'agitation d'au moins 15 W/m³ de volume de réacteur ou une énergie d'aération d'au moins 40 W/m³ de volume de réacteur.

13. Utilisation selon l'une quelconque des revendications 1 à 12, dans laquelle le processus MBBR comprend le mélange des supports dans le bioréacteur à l'aide de mélangeurs mécaniques immergés.

14. Utilisation selon l'une quelconque des revendications 1 à 13, dans laquelle des eaux usées purifiées lors du processus MBBR contiennent des substances nocives préjudiciables pour le support.

15. Utilisation selon l'une quelconque des revendications 1 à 14, dans laquelle la fonction du processus MBBR dépend de l'ajout de substances chimiques externes, telles que des sources externes de carbone.

16. Utilisation selon l'une quelconque des revendications 1 à 15, dans laquelle le processus MBBR est un traitement pour la production d'eau potable.
